# FASCICULE DE BREVET EUROPEEN

(11) **EP 0 839 149 B1**
(45) Date de publication et mention de la délivrance du brevet: **14.04.2004**
(21) Numéro de dépôt: 96926431.6
(22) Date de dépôt: 19.07.1996
(51) Int. Cl.: C07D 491/06, A61K 31/55

(54) **DERIVES DE LA GALANTHAMINE, LEUR PROCEDE DE PREPARATION, LEUR APPLICATION COMME MEDICAMENTS ET LES COMPOSITIONS PHARMACEUTIQUES LES RENFERMANT**
GALANTHAMIN-DERIVATE, VERFAHREN ZU IHRER HERSTELLUNG, IHRE VERWENDUNG ALS ARZMITTEL UND DIESE ENTHALTENDE PHARMAZEUTISCHE ZUSAMMENSETZUNG
GALANTHAMINE DERIVATIVES, PREPARATION METHOD THEREFOR, USE THEREOF AS DRUGS, AND PHARMACEUTICAL COMPOSITIONS CONTAINING SUCH DERIVATIVES

(30) Priorité: 19.07.1995 GB 9514821
(43) Date de publication de la demande: 06.05.1998
(73) Titulaire: CENTRE NATIONAL DE LA RECHERCHE SCIENTIFIQUE, 75016 Paris Cédex (FR)
(72) Inventeur: THAL, Claude, F-92330 Sceaux (FR); GUILLOU, Catherine, F-91190 Gif-sur-Yvette (FR); MARY, Aude, F-91210 Draveil (FR); RENKO, Dolor, F-91190 Gif-sur-Yvette (FR); POTIER, Pierre, F-75007 Paris (FR); CHRISTEN, Yves, F-75016 Paris (FR)
(74) Mandataire: Texier, Christian
(86) Numéro de dépôt international: PCT/FR1996/001139
(87) Numéro de publication internationale: WO 1997/003987

(56) Documents cités:
- EP-A- 0 653 427
- WO-A-88/08708
- CHEMICAL ABSTRACTS, vol. 115, no. 1, 8 Juillet 1991 Columbus, Ohio, US; abstract no. 5115u, SHIGERU KOBAYASHI ET AL: "Alkaloid N-oxides from Lycoris sanguinea " page 5113; XP002014290 & PHYTOCHEMISTRY, vol. 30, no. 2, 1991, pages 675-677,
- HELVETICA CHIMICA ACTA, vol. 77, 1994, BASEL CH, pages 1611-1615, XP002014289 R. MATUSCH ET AL : "146. Bildung, Kristallstruktur und absolute Konfiguration von (-)-N-(Chloromethyl)gananthaminium-chlorid "

## Description

La présente invention concerne de nouveaux dérivés de la galanthamine, un procédé pour leur préparation, des compositions pharmaceutiques les contenant et leur utilisation notamment en tant qu'inhibiteurs de cholinestérase.

Un certain nombre d'inhibiteurs de cholinestérase sont cliniquement utilisés pour antagoniser le blocage neuromusculaire induit par les relaxants musculaires non dépolarisants afin de pallier les diverses anomalies liées à la transmission cholinergique telle que le maintien de la force du muscle chez les malades atteints de myasthénie gravis ou le traitement du glaucome. Depuis quelques années, les études pharmacologiques des inhibiteurs de cholinestérase se sont très fortement développées, conduisant à la mise au point de médicaments comme la tacrine. Ce composé atténue les symptômes de la maladie d'Alzheimer qui résulte d'un processus neurodégénératif progressif caractérisé par une perte de mémoire à court et long terme, des fonctions cognitives et des performances intellectuelles. Parmi ces inhibiteurs, la galanthamine de formule 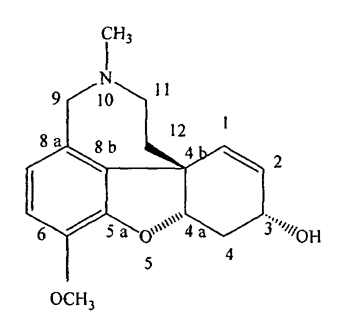 est bien connue. C'est un alcaloïde de la famille des Amaryllidacées qui fût isolé du perce-neige *Galantus Nivalis.* Cet alcaloïde est un inhibiteur réversible de l'acétylcholinestérase et est généralement utilisé dans le traitement des maladies neurodégénératives et plus récemment les démences séniles de type Alzheimer. Le bromhydrate de galanthamine est cliniquement utilisé sous le nom commercial de Nivaline®.

L'invention a donc pour objet les produits de formule générale Ia 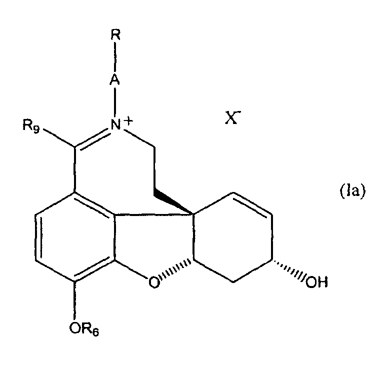 dans laquelle
A représente un groupe alkylène linéaire ou ramifié, saturé ou insaturé, contenant de 1 à 12 atomes de carbone ;
R représente un atome d'hydrogène ou un groupe de formule -NR'R" ou -N⁺R'R''R'''
dans laquelle
R' et R" représentent, indépendamment un atome d'hydrogène ; un radical cyano ; un radical alkyle comprenant de 1 à 12 atomes de carbone ; un radical aryle lié à un groupe alkyle comprenant de 1 à 12 atomes de carbone, le radical aryle étant choisi parmi les radicaux phényle, naphtyle, thiényle, furyle, pyrrolyle, imidazolyle, pyrazolyle, isothiazole, thiazole, isoxazolyle, oxazole, pyridyle, pyrazyle, pyrimidyle, benzothiényle, benzofuryle et indolyle ; ou R' et R" sont liés entre eux et forment avec l'atome d'azote auquel ils sont rattachés un hétérocycle choisi parmi les radicaux pyrrole, imidazole, isothiazole, thiazole, isoxazole, oxazole, pyridine, pyrazine, pyrimidine, pyridazine, indole, isoindole, indazole, quinoline, isoquinoline, phthalazine, quinazoline, pyrrolidine, imidazolidine, pyrrazolidine, piperidine, piperazine, morpholine, thiazolidine et phthalimide, R''' représente, un radical cyano, un radical alkyle comprenant de 1 à 12 atomes de carbone,
R₆ représente un atome d'hydrogène ou un radical de formule -A-R dans laquelle A et R ont la signification indiquée ci-dessus ;
R₉ représente un atome d'hydrogène ou un radical de formule R'₉ dans laquelle R'₉ représente un radical alkyle linéaire ou ramifié, contenant de 1 à 12 atomes de carbone ou un radical alcènyle linéaire ou ramifié, contenant de 1 à 12 atomes de carbone ;
X représente un anion pharmaceutiquement acceptable.

L'anion X⁻ pharmaceutiquement acceptable peut être formé par les acides organiques ou inorganiques, tels que les acides chlorhydrique, bromhydrique, sulfurique, phosphorique, acétique, trifluoroacétique, citrique, propionique, malonique, succinique, fumarique, tartrique, cinnamique, méthanesulfonique et p-toluène-sulfonique.

Le produit de formule Ia tel que défini ci-dessus est, à certaines valeurs de pH, en équilibre avec le produit correspondant de formule Ib 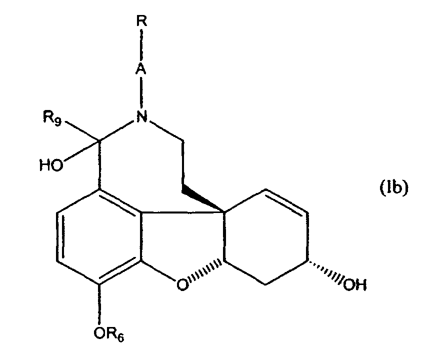 dans laquelle R, A, R₆ et R₉ ont la signification indiquée ci-dessus, selon le schéma suivant :

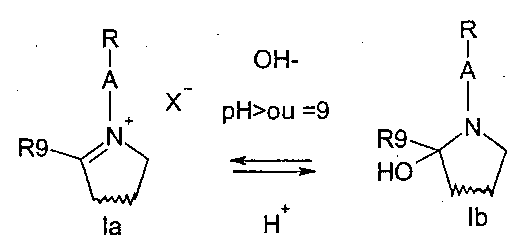

L'existence de cet équilibre, selon la valeur du pH, peut être intéressante pour franchir les barrières biologiques selon le mode d'administration utilisé.

L'invention a donc également pour objet des produits de formule Ib telle que définie ci-dessus ainsi que les sels de ces produits.

Dans les expressions indiquées ci-dessus, l'expression halo représente un radical fluoro, chloro, bromo ou iodo, de préférence bromo.

Les terme alkylène saturé désigne une chaîne hydrocarbonée, linéaire (polyméthylène) ou ramifiée, comprenant de 1 à 12 atomes de carbone. Ainsi le terme alkylène peut désigner les radicaux méthylène, éthylène, propylène, butylène, pentylène (pentaméthylène), hexylène (hexaméthylène), heptylène, octylène, nonanylène, décanylène, undécanylène et dodécanylène.

Par groupe alkylène insaturé, on entend des groupements comprenant une ou plusieurs doubles liaisons et/ou une ou plusieurs triples liaisons.

On comprend en particulier les groupes comportant une ou plusieurs doubles liaisons et en particulier les groupes alkénylènes comportant une double liaison, tels que le groupe vinylène (ou éthénylène), le groupe propénylène. On comprend également les groupes comportant une ou plusieurs triples liaisons et en particulier les groupes alkynylènes comportant une triple liaison tel que le groupe éthynylène ou propynylène.

Ces différents groupes peuvent également être ramifiés. On peut citer, par exemple, le groupe éthyléthylène, le groupe de formule CH₃-CH-CH₂- ou le groupe 4-propyl-2-penténylène.

Le terme alkyle désigne un radical alkyle linéaire ou ramifié comprenant de 1 à 12 atomes de carbone. De préférence, le terme alkyle représente un radical alkyle ayant de 1 à 6 atomes de carbone linéaire ou ramifié et en particulier les radicaux méthyle, éthyle, propyle, isopropyle, butyle, isobutyle, sec-butyle et tert-butyle, pentyle, isopentyle, hexyle, isohexyle.

Le terme alkényle désigne un radical alkényle linéaire ou ramifié comprenant 1 à 12 atomes de carbone. De préférence, le terme alkényle représente un radical alkényle ayant 1 à 6 atomes de carbone linéaire ou ramifié et en particulier les radicaux vinyle, allyle, propényle, butényle, pentényle ou hexényle.

Le terme haloalkyle désigne de préférence un radical alkyle tel que défini ci-dessus et substitué par un ou plusieurs atomes d'halogène tel que défini ci-dessus comme, par exemple, bromoéthyle, trifluorométhyle, trifluoroéthyle ou encore pentafluoroéthyle.

Le terme alkylthio désigne les radicaux dans lesquels le radical alkyle a la signification indiquée ci-dessus. De préférence, le terme alkylthio représente un radical méthylthio, éthylthio, propylthio, butylthio ou pentylthio.

Les radicaux alkoxy désignent les radicaux dont le radical alkyle a la signification indiquée ci-dessus. On préfère les radicaux méthoxy, éthoxy, isopropyloxy ou tert-butyloxy.

L'expression cycloalkyle désigne un cycloalkyle, saturé ou insaturé, de 3 à 7 atomes de carbone. Les radicaux cycloalkyles saturés peuvent être choisis parmi les radicaux cyclopropyle, cyclobutyle, cyclopentyle, cyclohexyle ou cycloheptyle. Les radicaux cycloalkyles insaturés peuvent être choisis parmi les radicaux cyclobutène, cyclopentène, cyclohexène, cyclopentanediène, cyclohexadiène.

L'expression amino éventuellement substitué par un ou plusieurs radicaux alkyles identiques ou différents représente le radical amino éventuellement substitué par un ou plusieurs radicaux alkyles tels que définis ci-dessus. De préférence, cette expression désigne le radical amino, les radicaux monoalkylamino tels que méthylamino ou éthylamino, ou dialkylamino tels que diméthylamino ou diéthylamino.

L'expression hétérocycle désigne un hétérocycle saturé ou insaturé, monocyclique ou polycyclique, éventuellement substitué et contenant de 3 à 9 atomes de carbone et au moins un atome d'azote. L'hétérocycle peut contenir plusieurs hétéroatomes identiques ou différents. De préférence, les hétéroatomes sont choisis parmi l'oxygène, le soufre ou l'azote. Des exemples d'hétérocycle sont les radicaux pyrrole, imidazole, isothiazole, thiazole, isoxazole; oxazole, pyridine, pyrazine, pyrimidine, pyridazine, indole, isoindole, indazole, quinoléine, isoquinoléine, phthalazine, quinazoline, pyrrolidine, imidazolidine, pyrrazolidine, pipéridine, pipérazine, morpholine, thiazolidine ou phthalimide.

L'expression acyle désigne un radical acyle ayant de 1 à 6 atomes de carbone tel que, par exemple, le radical formyle, acétyle, propionyle, butyryle, pentanoyle, hexanoyle, acryloyle, crotonoyle ou benzoyle.

L'expression aryle représente un radical insaturé, constitué d'un cycle ou de cycles condensés ; chaque cycle peut éventuellement contenir un ou plusieurs hétéroatomes identiques ou différents choisis parmi le soufre, l'azote ou l'oxygène. Des exemples de radical aryle sont les radicaux phényle, naphtyle, thiényle, furyle, pyrrolyle, imidazolyle, pyrazolyle, isothiazole, thiazole, isoxazolyle, oxazolyle, pyridyle, pyrazyle, pyrimidyle, benzothiényle, benzofuryle et indolyle.

Plus particulièrement, l'invention a pour objet les produits décrits ci-après dans les exemples, en particulier les produits répondant aux formules suivantes :
- le méthanesulfonate de galanthaminium ;
- le trifluoroacétate de 10-N-déméthyl-10-N-(4'-phthalimidobutyl)-galanthaminium ;
- le trifluoroacétate de 10-N-déméthyl-10-N-(6'-phthalimidohexyl)-galanthaminium ;
- le trifluoroacétate de 10-N-déméthyl-10-N-(8'-phthalimidooctyl)-galanthaminium ;
- le bromohydrate de 10-N-déméthyl-10-N-(10'-phthalimidodécyl)-galanthaminium ;
- le bromohydrate de 10-N-déméthyl-10-N-(12'-phthalimidododécyl)-galanthaminium ;
- le bromohydrate de 6-O-déméthyl-6-O-(8'-phthalimidooctyl)-galanthaminium ;
- le bromohydrate de 6-O-déméthyl-6-O-(4'-phthalimidobutyl)-galanthaminium ;
- le bromohydrate de 6-O-déméthyl-6-O-(10'-phthalimidodécyl)-galanthaminium ;
- le bromohydrate de 6-O-déméthyl-6-O-(12'-phthalimidododécyl)-galanthaminium ;
- le bromohydrate de 10-N-déméthyl-10-N-(6'-pyrrolohexyl)-galanthaminium.

L'invention a également pour objet un procédé de préparation des produits de formules générales Ia et Ib telles définies ci-dessus, caractérisé en ce que
a) soit l'on oxyde directement un composé de formule générale (1a) 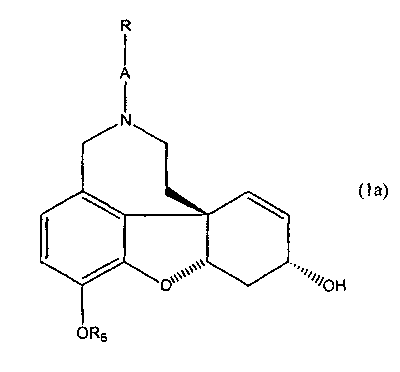 dans laquelle R, A et R₆ ont la signification indiquée ci-dessus ;
b) soit l'on transforme le composé de formule (1a) telle que définie ci-dessus, en son N-oxyde de formule (2a) 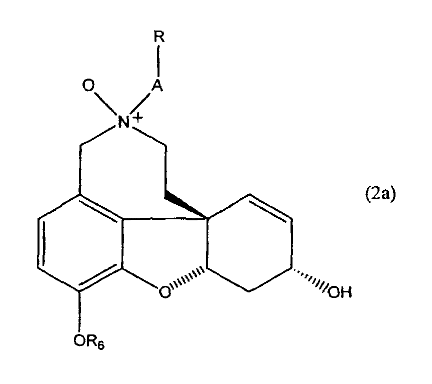
dans laquelle R, A et R₆ ont la signification indiquée à la revendication 1, produit de formule (2a) que l'on fait réagir avec un anhydride d'acide, sous atmosphère inerte, dans un solvant inerte, à une température comprise entre 0°C et la température ambiante,
pour obtenir un produit de formule Ia ou Ib dans laquelle R₉ représente un atome d'hydrogène, et
si le produit de formule Ia ou Ib dans laquelle R₉ représente R'₉ est recherché,
l'on traite le produit correspondant ainsi obtenu de formule Ia dans laquelle R₉ représente un atome d'hydrogène, avec un produit représentant une fonction nucléophile de formule R'₉Y dans laquelle R'₉ a la signification indiquée ci-dessus et
Y représente un radical approprié, pour obtenir le composé de formule (1b) 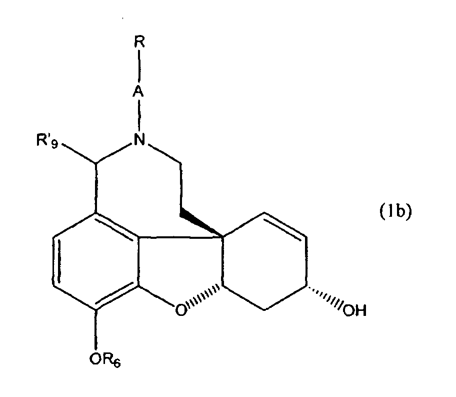 dans laquelle R, A, R₆ et R'₉ ont la signification indiquée ci-dessus, puis
a) soit l'on oxyde directement le composé de formule générale (1b),
b) soit l'on transforme le composé de formule générale (1b) en son N-oxyde de formule (2b) 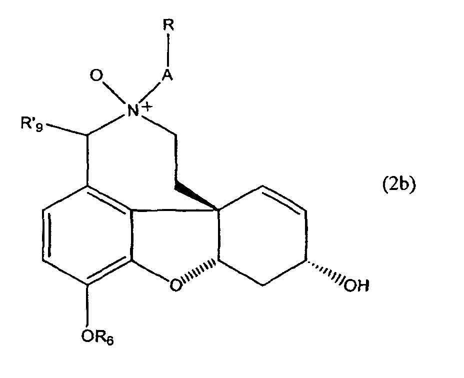
dans laquelle R, A, R₆ et R'₉ ont la signification indiquée ci-dessus, produit de formule (2b) que l'on fait réagir avec un anhydride d'acide, sous atmosphère inerte, dans un solvant inerte, à une température comprise entre 0°C et la température ambiante,
pour obtenir un produit de formule la ou Ib dans laquelle R₉ représente R'₉.

Les produits de l'invention dans lequel R₉ représente un atome d'hydrogène, peuvent donc être obtenus directement par oxydation du composé (1a) ou indirectement à partir de ce même composé de formule (1a). Les produits de l'invention dans lequel R₉ représente R'₉, peuvent également être obtenus directement par oxydation du composé de formule (1b) ou indirectement à partir de ce même composé de formule (1b).

L'oxydation directe peut être conduite selon les méthodes d'oxydation d'amines, connues de l'homme de l'art. Ainsi, l'oxydation peut être conduite sous atmosphère inerte, à une température comprise entre 10 et 30°C, dans un solvant aprotique tel que, par exemple, le tétrachlorure de carbone, en présence de N-bromosuccinimide et d'azodiisobutyronitrile (AIBN). L'oxydation peut également être conduite en présence d'iode dans un solvant protique polaire comme, par exemple, l'éthanol.

Le composé de formule (1a) ou (1b) est transformé en son N-oxyde correspondant de formule (2a) ou (2b) respectivement, selon une méthode classique de préparation de N-oxyde telle que, par exemple, par réaction avec un peracide dans des conditions douces. La réaction du composé de formule (2a) ou (2b) avec un anhydride d'acide, peut être réalisée dans un solvant inerte à une température comprise entre 0°C et la température ambiante ; elle est réalisée de préférence avec l'anhydride trifluoroacétique dans du dichlorométhane.

Le composé de formule (1b) est obtenu à partir du produit correspondant de formule Ia dans laquelle R₉ représente un atome d'hydrogène, en faisant réagir ce dernier avec un produit de formule R'₉Y et représentant une fonction nucléophile selon les méthodes classiques d'addition nucléophiles sur les fonctions imminiums, méthodes connues de l'homme de l'art ; dans ce produit de formule R'₉Y, R'₉ a la signification indiquée ci-dessus et Y peut représenter un élément métallé comme dans le cas des magnésiens, lithiens et des alkylstannylés ou Y représente respectivement MgHalogène, Li, Sn(Y')₃ dans lequel Y' représente un radical alkyle de 1 à 6 atomes de carbone, et de préférence le radical méthyle, butyle ou allyle.

Les composés de formule (1) dans laquelle R représente un atome d'hydrogène, sont décrits dans le brevet européen EP 236684.

Les composés de formule (1a) dans laquelle R représente un groupe de formule -NR'R" ou -N^{⊕}R'R''R''' peuvent être obtenus à partir de la galanthamine selon le schéma réactionnel 1 ci-dessous. Les réactions de O-déméthylation, d'addition d'un composé de formule R-A-X dans laquelle R et A sont tels que définis ci-dessus et X représente un atome d'halogène, ou d'oxydation telles que définies dans le schéma réactionnel 1, sont mises en oeuvre selon les méthodes classiques connues de l'homme de l'art. De plus, le composé de formule (1a) dans laquelle R représente un groupe phthalimido, peut être utilisé comme composé de départ pour la synthèse d'un composé de formule (1a) dans laquelle -NR'R" représente -NH₂. Ledit composé (1a) dans lequel -NR'R" représente -NH₂ peut lui-même également être utilisé comme composé de départ pour la synthèse des composés (1) dans lesquels -NR'H" représente un groupe amino substitué.

L'invention a également pour objet, à titre de produits industriels nouveaux, et notamment à titre de produits industriels nouveaux destinés à la préparation des produits de formule Ia ou Ib, les produits de formules (1a), (1b) et (2b) telles que décrites ci-dessus, dans laquelle R représente un groupe de formule -NR'R" ou -N⊕R'R''R''' telle que définie ci-dessus.

Les composés de la présente invention sont des inhibiteurs de cholinestérase. Cette propriété les rend aptes à une utilisation pharmaceutique. Ils peuvent ainsi être utilisés dans différentes applications thérapeutiques. Ainsi, les composés de l'invention peuvent être utilisés pour le traitement des maladies neurodégénératives mais également les démences séniles de type Alzheimer.

On trouvera ci-après, dans la partie expérimentale, une illustration des propriétés pharmacologiques des composés de l'invention.

La présente demande a également pour objet, à titre de médicaments, les produits de formule Ia ou Ib telle que définie ci-dessus, ainsi que les sels d'addition avec les acides minéraux ou organiques pharmaceutiquement acceptables desdits produits de formule Ib, ainsi que les compositions pharmaceutiques contenant, à titre de principe actif, l'un au moins des médicaments tels que définis ci-dessus.

L'invention concerne ainsi des compositions pharmaceutiques contenant à titre de principe actif, l'un au moins des médicaments tels que définis ci-dessus, en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols.

Les compostions selon l'invention peuvent être administrées par les voies classiques d'administration telle que orale, parentérale ou intramusculaire.

L'invention a également pour objet l'utilisation des produits de formule la (ou Ib) telle que définie ci-dessus, pour la préparation de médicaments destinés à traiter les maladies neurodégénératives ainsi que de médicaments destinés à traiter les démences séniles.

Les exemples suivants sont présentés pour illustrer les procédures ci-dessus et ne doivent en aucun cas être considérés comme une limite à la portée de l'invention.

### PARTIE EXPERIMENTALE :

### Préparation 1 : norgalanthamine

On agite une solution de 1,73 g (5,7 mmoles) de N-oxyde de galanthamine et de 3,17 g (11,4 mmoles, 2 éq) de FeSO₄-7H₂O dans 100 ml de méthanol pendant 1 heure 30 à 10°C, sous argon. Après évaporation du solvant, le résidu est repris dans du dichlorométhane, traité par une solution aqueuse saturée en hydrogénocarbonate de sodium et extrait trois fois avec du dichlorométhane. Les phases organiques sont rassemblées et lavées avec une solution aqueuse saturée en chlorure de sodium, séchées sur sulfate de sodium et évaporées. Le produit brut de la réaction est purifié par flash chromatographie sur gel de silice en utilisant le mélange dichlorométhane / méthanol (80 : 20) comme éluant. La fraction la moins polaire donne la galanthamine et la fraction la plus polaire donne majoritairement la norgalanthamine sous forme d'une mousse blanche (rendement = 76 %).

RMN-¹H (300 MHz-CD₃ OD): 6,71 (1H, d, J=8 Hz, H₇); 6,63 (1H, d, J=8 Hz, H₈); 6,12 (1H, d, J=10 Hz, H₁); 5,91 (1H, dd, J₁ =10 Hz, J₂ =5 Hz, H₂); 4,53 (1H, s large, H₄ₐ); 4,14 (1H, t large, J=5 Hz, H₃); 4,06 (1H, d J=15 Hz, H_{9.α}); 3,89 (1H, d, J=15 Hz, H_{9.β}); 3,79 (3H, s, OCH₃); 3,25 (2H, m, H₁₁); 2,49 (1H, dm, J=16 Hz, H_{4.α}); 2,24 (1H, ddd, J1 =16 Hz, J2 =5 Hz, J3 =3 Hz, H_{4.β}); 1,84 (2H, m, H₁₂).

### Préparation 2 : 10-N-deméthyl-10-N-(4'-phthalimidobutyl)-galanthamine

On ajoute 2,11 g (7,5 mmoles, 1,2 éq) de N-(4-bromobutyl)phthalimide et 1,7 ml de triéthylamine à une solution de 1,70 g (6,2 mmoles) de norgalanthamine dans 50 ml d'acétonitrile. Le mélange réactionnel est agité puis porté au reflux pendant 20 heures. Après évaporation du solvant, le résidu est repris dans 10 ml de dichlorométhane et 10 ml d'eau. Une solution d'acide chlorhydrique 1 N est additionnée jusqu'à pH 4-5. La phase organique est récupérée, lavée trois fois avec une solution saturée aqueuse de carbonate de sodium, séchée sur du sulfate de sodium, filtrée et évaporée. Le produit brut est purifié par chromatographie sur gel de silice avec un mélange dichlorométhane / méthanol (90/10) comme éluant pour donner le produit final sous forme d'une huile jaune (rendement = 81 %).

RMN-¹H (300 MHz-CD₃ OD): 7,83-7,72 (4H, m, phthalimido); 6,61 (1H, d, H₇); 6,54 (1H, d, H₈); 6,14 (1H, d, H₁); 5,91 (1H, dd, H₂); 4,53 (1H, large, s, H₄ₐ); 4,15 (1H, d, H_{9.α}); 4,13 (1H, large, t, H₃); 3,77(1H, d, H_{9.β}); 3,75 (3H, s, OCH₃); 3,65 (2H, t, H_{4'}); 3,34 (1H, large t, H_{11.α}); 3,14 (1H, dm, H_{11.β}); 2,57-2,44 (3H, m, H_{1'}, H_{4.α}); 2,14-2,01 (2H, m, H_{12.α}, H_{4.β}); 1,63 (2H, m, H_{2'}); 1,59-1,48 (3H, m, H_{3'}, H_{12.β}).

### Préparation 3 : 10-N-deméthyl-N-(6'-phthalimidohexyl)-galanthamine

La réaction est conduite selon la méthode décrite dans l'exemple de préparation 2, en utilisant du N-(6-bromohexyl)-phthalimide à la place du N-(4-bromobutyl)-phthalimide (rendement = 80 %).

RMN-¹H (300 MHz-CDCl₃): 7,85-7,82 (2H, m, phthalimido); 7,72-7,69 (2H, m, phthalimido); 6,66 (1H, d, H₇); 6,61 (1H, d, H₈); 6,09 (1H, d; H₁); 6,00 (1H, dd, H₂); 4,61 (1H, large s, H₄ₐ); 4,14 (1H, large t, H₃); 4,12 (1H, d, H_{9.α}); 3,83 (3H, s, OCH₃); 3,80 (1H, d, H_{9.β}); 3,66 (2H, t, H_{6'}); 3,35 (1H, large t, H_{11.α}); 3,16 (1H, large d, H_{11.β}) 2,68 (1H, dm, H_{4.β}); 2,52-2,39 (2H, m, H_{1'}); 2,10-1,96 (2H, m, H_{12.α}, H_{4.β}); 1,72-1,60 (2H, m, H_{5'}); 1,54-1,41(3H, m, H_{2'}, H_{12.β}); 1,38-1,29(4H, m, H_{4'}, H_{3'}).

### Préparation 4 : N-oxyde de 10-N-deméthyl-10-N-(4'-phthalimidobutyl)-galanthamine

On ajoute 116 mg (0,67 mmole, 1,1 éq) d'acide métachloroperbenzoïque à 70 % à une solution de 203 mg (0,43 mmole) de 10-N-deméthyl-10-N-(4'-phthalimidobutyl)-galanthamine dans 10 ml de dichlorométhane anhydride. Le mélange réactionnel est agité à température ambiante sous atmosphère inerte pendant 2 heures et demie. Le solvant est évaporé sous vide et le résidu purifié par flash chromatographie sur gel de silice en utilisant un mélange de dichlorométhane / méthanol (80/20) comme éluant pour fournir le produit pur sous la forme d'une mousse blanche (86 %).

RMN-¹H (250 MHz-CD₃ OD): 7,82-7,70 (4H, m, phthalimido); 6,80-6,55 (2H, s, H₇, H₈); 6,08 (2H, s, H₁, H₂); 4,84 (1H, large d, H_{9.α}); 4,69 (1H, large s, H₄ₐ); 4,41 (1H, d, H_{9.β}); 4,18 (1H, t, H₃); 4,11-3,89 (1H, m, H_{11.α}); 3,85-3,78 (1H, m, H_{11.β}); 3,82 (3H, s, OCH₃); 3,64 (2H, t, H_{4'}); 3,15-3,05 (2H, m, H_{1'}); 2,71 (1H, dm, H_{4.α}); 2,05 (1H, ddd, H_{4.β}); 1,97-1,83 (2H, m, H₁₂); 1,75-1,63 (4H, m, H_{2'}, H_{3'}).

### Préparation 5 : 10-N-deméthyl-10-N-(8'-phthalimidooctyl)-galanthamine

La réaction est conduite selon la méthode décrite dans l'exemple de préparation 2, en utilisant du N-(8-bromooctyl)-phthalimide à la place du N-(4-bromobutyl)-phthalimide (rendement = 82 %).

RMN-¹H (300 MHz-CDCₗ₃): 7,84 (2H, m, H_{ar meta}); 7,71 (2H, m, H_{ar ortho}); 6,66 (1H, d, J=8 Hz, H₇); 6,61 (1H, d, J=8 Hz, H₈); 6,08 (1H, d, J=10 Hz, H₁); 6,00 (1H, dd, J₁ =10 Hz, J₂ =4.5 Hz, H₂); 4,61 (1H, large s, H₄ₐ); 4,14 (1H, large t, H₃); 4,13 (1H, d, J=15 Hz, H_{9.α}); 3,83 (3H, s, OCH₃); 3,80 (1H, d, J=15 Hz, H_{9.β}); 3,66 (2H, t, J=7 Hz, H_{8'}); 3,35 (1H, large t, J₁ =15 Hz, J₂ =13 Hz, H_{11.α}); 3,17 (1H, large d, J=15 Hz, H_{11.β}); 2,68 (1H, dm, J=15.5 Hz, H_{4.α}); 2,46 (2H, m, H_{1'}); 2,40 (1H, large s, OH); 2,08-1,97 (2H,m, H_{12.α}, H_{4.β}); 1,65 (2H, m, H_{7'}); 1,54-1,43 (3H, m, H_{12.β}, H_{2'}); 1,31 (4H, large s, H_{6'}, H_{3'}); 1,26 (4H, m, H_{4'}, H_{5'}).

### Préparation 6 : 10-N-deméthyl-10-N-(10'-phthalimidodécyl)-galanthamine

La réaction est conduite selon la méthode décrite dans l'exemple de préparation 2, en utilisant du N-(10-bromodécyl)-phthalimide à la place du N-(4-bromobutyl)-phthalimide (rendement = 84 %).

RMN-¹H (300 MHz-CDCl₃): 7,82 (2H, m, H_{ar meta}); 7,70 (2H, m, H_{ar ortho}); 6,66 (1H, d, J=8 Hz, H₇); 6,61 (1H, d, J=8 Hz, H₈); 6,09 (1H, d, J=10 Hz, H₁); 5,99 (1H, dd, J₁ =10 Hz, J₂ =5 Hz, H₂); 4,61 (1H, large s, H₄ₐ); 4,14 (1H, large t, H₃); 4,13 (1H, d, J=15 Hz, H_{9.α}); 3,82 (3H, s, OCH₃); 3,81 (1H, d, J=15 Hz, H_{9.β}); 3,66 (2H, t, J=7 Hz, H_{10'}); 3,35 (1H, large t, J1 =15 Hz, J2 =13 Hz, H_{11.α}); 3,17 (1H, large d, J=15 Hz, H_{11.β}); 2,67 (1H, dm, J=14 Hz, H_{4.α}); 2,47 (2H, m, H_{1'}); 2,09-1,97 (2H, m, H_{12.α}, H_{4.β}); 1,66 (2H, m, H_{9'}); 1,54-1,46 (3H, m, H_{12.β}, H_{2'}); 1,31 (2H, large s, H_{8'}); 1,25 (10H, large s, H_{3'}, H_{4'}, H_{5'}, H_{6'}, H_{7'}).

### Préparation 7 : 10-N-deméthyl-10-N-(12'-phthalimidododécyl)-galanthamine

La réaction est conduite selon la méthode décrite dans l'exemple de préparation 2, en utilisant du N-(12-bromododécyl)-phthalimide à la place du N-(4-bromobutyl)-phthalimide (rendement = 81 %).

RMN-¹H (300 MHz-CDCl₃): 7,84 (2H, m, H_{ar meta}); 7,70 (2H, m, H_{ar ortho}); 6,66 (1H, d, J=8 Hz, H₇); 6,61 (1H, d, J=8 Hz, H₈); 6,09 (1H, d, J=10 Hz, H₁); 6,00 (1H, dd, J₁ =10 Hz, J₂ =5 Hz, H₂); 4,61 (1H, large s, H₄ₐ); 4,14 (1H, large t, H₃); 4,13 (1H, d, J=15 Hz, H_{9.α}); 3,83 (3H, s, OCH₃); 3,82 (1H, d, J=15 Hz, H_{9.β}); 3,67 (2H, t, J=7 Hz, H_{12'}); 3,36 (1H, large t, J1 =15 Hz, J2 =13 Hz, H_{11.α}); 3,18 (1H, large d, J=15 Hz, H_{11.β}); 2,68 (1H, dm, J=15.5 Hz, H_{4.α}); 2,46 (3H, m, OH, H_{1'}); 2,05-1,96 (2H, m, H_{12.α}, H_{4.β}); 1,66 (2H, m, H_{11'}); 1,54-1,45 (3H, m, H_{12.β}, H_{2'}); 1,31 (2H, m, H_{10'}); 1,24 (14H, large s, H_{3'}, H_{4'}, H_{5'}, H_{6'}, H_{7'}, H_{8'}, H_{9'}).

### Préparation 8 : 6-O-deméthylgalanthamine

On additionne 8 ml (7,96 mmoles, 4,5 éq) de L-sélectride 1M dans le tétrahydrofuranne à une solution de 507 mg (1,77 mmoles) de galanthamine dans 20 ml de tétrahydrofuranne. On chauffe le mélange à 67°C sous argon, pendant 20 heures. On refroidit le milieu réactionnel à 0°C, on le dilue avec 25 ml d'acétate d'éthyle, puis on ajoute lentement 25 ml d'eau. On sépare la phase aqueuse de la phase organique et on la concentre sous vide. On purifie le produit brut par flash chromatographie sur gel de silice avec un mélange dichlorométhane / méthanol / ammoniaque (90 : 9 : 1) pour obtenir un solide beige qui, après recristallisation dans l'acétone, fournit la 6-O-deméthylgalanthamine (rendement = 95 %).

RMN-¹H (250 MHz-CD₃ OD): 8,06 (H, s, H₉); 7,24 (1H, d, J=8.5 Hz, H₈); 6,43(1H, d, J=8.5 Hz, H₇); 6,01 (1H, dd, J₁ =10 Hz, J₂ =5 Hz, H₂); 5,78 (1H, d, J=8.5 Hz, H₁); 4,53 (1H, large s, H₄ₐ); 4,15-4,05 (2H, m, H₃, H_{11.α}); 3,81 (1H, dm, J=16 Hz, H_{11.β}); 3,57 (3H, s, NCH₃); 2,56 (1H, dm, J=15.5 Hz, H_{4.α}); 2,18-2,06 (3H, m, H_{4.β}, H₁₂).

### Préparation 9 : 6-O-deméthyl-6-O-(4'-phthalimidobutyl)-galanthamine

Une solution de 79 mg (0,29 mmole) de 6-O-deméthylgalanthamine et de 94 mg (0,29 mmole) de carbonate de césium dans 3 ml de diméthylformamide distillé est agitée à température ambiante pendant 30 minutes. 90 mg (0,32 mmole, 1,1 éq) de N-(4-bromobutyl)-phthalimide sont ensuite additionnés et le mélange est chauffé au reflux pendant 2 heures. Après évaporation du solvant sous vide, le résidu est repris par 3 x 20 ml de dichlorométhane et lavé avec 50 ml d'une solution aqueuse saturée en chlorure de sodium. Les phases organiques sont réunies, séchées sur sulfate de sodium, filtrées et évaporées. Le produit brut est purifié par chromatographie sur couche épaisse avec comme mélange d'élution : dichlorométhane 90 / méthanol 10 / ammoniaque vapeurs (rendement = 70 %).

RMN-¹H (300 MHz-CDCl₃): 7,89 (2H, m, H_{ar ortho}); 7,77 (2H, m, H_{ar meta}); 6,64 (1H, d, J=8 Hz, H₇); 6,58 (1H, d, J=8 Hz, H₈); 6,05 (1H, d, J=10 Hz, H₁); 6,02 (1H, dd, J₁ =10 Hz, J₂ =4.5 Hz, H₂); 4,57 (1H, large s, H₄ₐ); 4,11 (1H, large t, J=4.5 Hz H₃); 4,10 (1H, d, J=15 Hz, H_{9.α}); 3,95 (2H, m, H₄); 3,69 (1H, d, J=15 Hz, H_{9.β}); 3,68 (2H, t, J=7 Hz, H_{1'}); 3,28 (1H, large t, J1 =14 Hz, H_{11.α}); 3,06 (1H, large d, J=14 Hz, H_{11.β}); 2,68 (1H, dm, J=16 Hz, H_{4.α}); 2,42 (3H, s, NCH₃); 2,11 (1H, dm, J=15 Hz, H_{12.α}); 1,99 (1H, ddd, J=15 Hz, H_{4.β}); 1,89-1,72 (3H, m, H_{2'}, H_{12.β}); 1,63 (2H, m, H_{3'}).

### Préparation 10 : 6-O-deméthyl-6-O-(8'-phthalimidooctyl)-galanthamine

En travaillant de la même manière que dans la préparation 9 mais en utilisant la N-(8-bromooctyl)phthalimide à la place de la N-(4-bromobutyl)phthalimide, on obtient le produit recherché (rendement = 45 %).

RMN-¹H (200 MHz-CDCl₃):7,83 (2H, m, H_{ar ortho}); 7,71 (2H, m, H_{ar meta}); 6,65 (1H, d, J=8 Hz, H₇); 6,58 (1H, d, J=8 Hz, H₈); 6,05 (1H, d, J=10.25 Hz, H₁); 5,98 (1H, dd, J₁ =10.25 Hz, J₂ =4.5 Hz, H₂); 4,59 (1H, large s, H₄ₐ); 4,12 (1H, large t, H₃); 4,08 (1H, d, J=15 Hz, H_{9.α}); 4,02 (2H, m, H_{8'}); 3,67 (1H, d, J=16 Hz, H_{9.β}); 3,66 (2H, t, J=6.5 Hz, H_{1'}); 3,28 (1H, large t, J₁ =14 Hz, J₂ =13 Hz, H_{11.α}); 3,04 (1H, m, J=14 Hz, H_{11.β}); 2,83 (1H, large s, OH); 2,68 (1H, dm, J=16 Hz, H_{4.α}); 2,39 (3H, s, NCH₃); 2,11 (1H, dm, J=13.5 Hz, H_{12.α}); 1,99 (1H, ddd, J=16 Hz, H_{4.β}); 1,75-1,53 (5H, m, H_{7'}, H_{2'}, H_{12.β}); 1,33 (8H, large s, H_{3'}, H_{6'}, H_{4'}, H_{5'}).

### Préparation 11 : 6-O-deméthyl-6-O-(10'-phthalimidodécyl)-galanthamine

En travaillant de la même manière que dans la préparation 9 mais en utilisant la N-(10-bromodécyl)phthalimide à la place de la N-(4-bromobutyl)phthalimide, on obtient le produit recherché (rendement = 48 %).

RMN-¹H (300 MHz-CDCl₃): 7,84 (2H, m, H_{ar meta}); 7,71 (2H, m, H_{ar ortho}); 6,65 (1H, d, J=8 Hz, H₇); 6,60 (1H, d, J=8 Hz, H₈); 6,05 (1H, d, J=10 Hz, H₁); 6,00 (1H, dd, J₁= 10.25 Hz, J₂ =5 Hz, H₂); 4,58 (1H, large s, H₄ₐ); 4,13 (1H, large t, H₃); 4,12 (1H, d, J=15.5 Hz, H_{9.α}); 4,01 (2H, t, J=6.5 Hz, H_{10'}); 3,67 (1H, d, J=15.5 Hz, H_{9.β}); 3,65 (2H, t, J=7 Hz, H_{1'}); 3,27 (1H, large t, J₁ =14.5 Hz, J₂ =13 Hz, H_{11.α}); 3,16 (1H, large d, J=14.5 Hz, H_{11.β}); 2,66 (1H, dm, J=16 Hz, H_{4.α}); 2,44 (1H, s, OH); 2,36 (3H, s, NCH₃); 2,07 (1H, dm, J=13 Hz, H_{12.α}); 1,99 (1H, dm, J=16 Hz, H_{4.β}); 1,74-1,53 (5H, m, H_{2'}, H_{9'}, H_{12.β}); 1,31 (12H, large s, H_{3'}, H_{4'}, H_{5'}, H_{6'},H_{7'}, H_{8'}).

### Préparation 12 : 6-O-deméthyl-6-O-(12'-phthalimidododécyl)-galanthamine

En travaillant de la même manière que dans la préparation 9 mais en utilisant la N-(12-bromododécyl)phthalimide à la place de la N-(4-bromobutyl)phthalimide, on obtient le produit recherché (rendement = 67 %).

RMN-¹H (300 MHz-CDCl₃): 7,85 (2H, m, H_{ar meta}); 7,70 (2H, m, H_{ar ortho}); 6,66 (1H, d, J=8 Hz, H₇); 6,59 (1H, d, J=8 Hz, H₈); 6,07 (1H, d, J=10 Hz, H₁); 5,99 (1H, dd, J₁ =10 Hz, J₂ =4.5 Hz, H₂); 4,60 (1H, large s, H₄ₐ); 4,14 (1H, large t, H₃); 4,13 (1H, d, J=15 Hz, H_{9.α}); 4,03 (2H, t, J=7 Hz, H_{12'}); 3,69 (1H, d, J=15 Hz, H_{9.β}); 3,66 (2H, t, J=7 Hz, H_{1'}); 3,29 (1H, large t, J₁ =15 Hz, J₂ =13 Hz, H_{11.α}); 3,18 (1H, large d, J=15 Hz, H_{11.β}); 2,67 (1H, dm, J=16 Hz, H_{4,α}); 2,46 (1H, s, OH); 2,38 (3H, s, NCH₃); 2,09 (2H, 1H, dm, J=14 Hz, H_{12.α}); 1,96 (1H, dm, J=16 Hz, H_{4.β}); 1,76-1,55 (5H, m, H_{2'}, H_{11'}, H_{12.β}); 1,35 (16H, large s, H_{3'}, H_{4'}, H_{5'}, H_{6'}, H_{7'}, H_{8'}, H_{9'}, H_{10'}).

### Préparation 13 : 10-N-deméthyl-10-N-(6'-aminohexyl)-galanthamine

A une solution de 1,67 g (3,32 mmoles) de 10-N-deméthyl-10-N-(6'-phthalimidohexyl)-galanthamine dans 50 ml d'éthanol 95°C sont ajoutés goutte à goutte 323 µl (2 éq, 6,64 mmoles) d'hydrazine hydratée. Le mélange est agité et porté au reflux pendant 18 heures. Une solution d'acide chlorhydrique 5N est additionnée jusqu'à pH 1. Le précipité blanc formé est éliminé par filtration, puis le filtrat est concentré. Le résidu est ensuite dissous dans 60 ml d'éthanol / eau (2 : 1) et du carbonate de sodium est additionné jusqu'à pH 10. La solution est extraite par 5 x 50 ml de dichlorométhane. La phase organique est séchée sur sulfate de sodium, filtrée et évaporée. Le résidu est purifié par flash chromatographie avec un mélange dichlorométhane / méthanol / ammoniaque (88 : 10 : 2) pour donner un solide blanc (rendement = 75 %).

RMN-¹H (300 MHz-CDCl₃): 6,66 (1H, d, J=8.25 Hz, H₇); 6,61 (1H, d, J=8.25 Hz, 6,09 (1H, d, J=10.25 Hz, H₁); 5,99 (1H, dd, J₁ =10.25 Hz, J₂ =4.75 Hz, H₂); 4,61 (1H, s large, H₄ₐ); 4,14 (1H, t large, J=4.75 Hz, H₃); 4,13 (1H, d, J=15.5 Hz, H_{9.α}); 3,83 (3H, s, OCH₃); 3,81 (1H, d, J=15.5 Hz, H_{11.β}); 3,35 (1H, large t, J=13 Hz, H_{11.α}); 3,17 (1H, large d, J=15 Hz, H_{11.β}); 2,68 (1H, dm, J=16 Hz, H_{4.α}); 2,66 (2H, t, J=7 Hz, H_{6'}); 2,55-2,40 (2H, m, H_{1'}); 2,09-2,04 (2H, m, H_{12α}); 2,01 (1H, m, H_{12.α}); 2,01 (1H, ddd, J₁ =16 Hz, J₂ =5 Hz, H_{4.β}); 1,77 (1H, large s, NH₂, OH); 1,55-1,37 (5H, m, H_{12.β}, H_{2'}, H_{5'}); 1,35-1,23 (4H, m, H_{4'}, H_{3'}).

### Préparation 14 : 10-N-deméthyl-10-N-(6'-pyrrolohexyl)-galanthamine

198 mg (2,40 mmoles, 12 éq) d'acétate de sodium sont additionnés à une solution de 75 mg (0,20 mmole) de 10-N-deméthyl-10-N-(6'-aminohexyl)-galanthamine dans 1,5 ml d'acide acétique (pH réaction = 7). Le mélange est chauffé à 70°C sous argon pendant 10 minutes puis 29 µl (0,22 mmole, 1,1 éq) de 2,5-diméthoxytétrahydrofuranne sont ajoutés. Au bout de 3 heures, 10 ml d'eau sont additionnés (pH 5) puis de petites quantités de carbonate de sodium pur ramener la solution à pH 9. La phase aqueuse est ensuite extraite avec 3 x 30 ml d'acétate d'éthyle. La phase organique est séchée sur sulfate de sodium, filtrée et évaporée. Le résidu est séparé par chromatographie sur couche épaisse avec un mélange dichlorométhane / méthanol / ammoniaque (90 : 10 : vapeurs) pour donner le composé sous forme d'une huile incolore (rendement = 79 %).

RMN-¹H (250 MHz-CDCl₃): 6,67 (1H, d, J=8.25 Hz, H₇); 6,64 (2H, t, J=2 Hz, H_{8'}, H_{11'}); 6,61(1H, d, J=8.25 Hz, H₈); 6,14 (2H, t, J=2 Hz, H_{9'}, H_{10'}); 6,09 (1H, dd, J₁ =10.5 Hz, J₂ =1 Hz, H₁); 6,00 (1H, dd, J₁ =10.5 Hz, J₂ =4.5 Hz, H₂); 4,61 (1H, s large, H₄ₐ); 4,15 (1H, t large, J=4.75 Hz, H₃); 4,14 (1H, d, J=15.5 Hz, H_{9.α}); 3,86 (2H, t, J=7 Hz, H_{6'}); 3,84 (3H, s, OCH₃); 3,81 (1H, d, J=15.5 Hz, H_{9.β}); 3,37 (1H, large t, J₁ =15 Hz, J₂ =13 Hz H_{11.α}); 3,16 (1H, dt, J₁ =15 Hz, J₂ =5, J₃ =3H_{11.β}); 2,73-2,65 (2H, m, H_{4.β}, OH); 2,56-2,38 (2H, m, H_{1'}); 2,10-2,05 (1H, m, H_{12.α}); 2,01 (1H, ddd, J₁ =16 Hz, J₂ =5.5 Hz, J₃ =2.5 Hz, H_{4.β}); 1,75 (2H, m, H_{5'}); 1,55-1,42 (3H, m, H_{12.β}, H_{2'}); 1,32-1,27 (4H, m, H_{4'}, H_{3'}).

### Exemple 1 : méthanesulfonate de galanthamine

On ajoute 87 mg (0,49 mmole, 1,3 éq) de N-bromosuccinimide à une solution de 127 mg (0,44 mmole) de galanthamine, dans 5 ml de tétrachlorure de carbone. Le mélange réactionnel est agité à température ambiante sous atmosphère inerte pendant 21 heures. On extrait alors par du dichlorométhane (3 x 30 ml) et par une solution aqueuse saturée de carbonate de sodium. La phase organique est séchée sur sulfate de sodium, filtrée et évaporée sous vide. Le produit brut ainsi obtenu est repris avec 5 ml de tétrahydrofuranne et 76 µl d'acide méthanesulfonique. Le mélange est agité à température ambiante, sous atmosphère inerte, pendant une heure. Après évaporation du solvant, le produit brut est lavé avec de l'acétate d'éthyle, puis purifié sur plaque préparative avec un mélange de dichlorométhane / méthanol (80/20) comme éluant pour obtenir le composé souhaité sous sa forme méthanesulfonate (rendement = 64 %).

RMN-¹H (250 MHz-CDCl₃): 8,67 (1H, s, H₉); 7,51 (1H, d, H₇); 7,05 (1H, d, H₈); 6,21 (1H, dd, H₂); 5,71 (1H, d, H₁); 4,87 (1H, large s, H₄ₐ); 4,54 (1H, dd, H₃); 4,30-4,10 (2H, m, H₁₁); 4,02 (3H, s, NCH₃); 3,88 (3H, s, OCH₃); 2,85 (1H, m, H_{4.α}); 2,30-2,10 (3H, m, H_{4.β}, H₁₂).

### Exemple 2 : trifluoroacétate de 10-N-deméthyl-10-N-(4'-phthalimidobutyl)-galanthaminium

On ajoute 308 µl (2,2 mmole, 6 éq) d'anhydride trifluoroacétique fraîchement distillé à une solution à 0°C de 178 mg (0,36 mmole) de N-oxyde de 10-N-deméthyl-10-N-(4'-phthalimidobutyl)galanthamine dans 10 ml de dichlorométhane. Le mélange réactionnel est agité pendant 3 heures à 0°C sous argon, puis on le laisse revenir à température ambiante. Le solvant est évaporé sous vide, puis le produit brut est purifié par chromatographie sur couche épaisse avec un mélange méthanol / dichlorométhane / ammoniaque (5 : 95 : vapeur) comme éluant pour obtenir 79 mg du composé Ib : 10-N-deméthyl-10-N-(4'-phthalimidobutyl)-9-hydroxygalanthamine. Après purification, ledit composé Ib est transformé en composé Ia correspondant, en présence d'acide trifluoroacétique (rendement = 44 %).

RMN-¹H (300 MHz-CDCl₃): 8,77 (1H, s, H₉); 7,85-7,72 (4H, m, phthalimido); 7,59 (1H, d, H₈); 7,02 (1H, d, H₇); 6,14 (1H, dd, H₂); 5,62 (1H, d, H₁); 4,81 (1H, large s, H₄ₐ); 4,38 (1H, large t, H₃); 4,27-4,05 (2H, m, H₁₁); 3,98 (3H, s, OCH₃); 3,77 (2H, large t, H_{4'}); 2,78 (1H, dm, H_{4.α}); 2,26 (2H, large t, H_{1'}); 2,22 (1H, m, H_{4.β}); 2,13 (1H, ddd, H_{12.α}); 2,00 (2H, m, H_{2'}); 1,88-1,66 (3H, m, H_{3'}, H_{12.β}).

### Exemple 3 : trifluoroacétate de 10-N-deméthyl-10-N-(6'-phthalimidohexyl)-galanthaminium

La réaction est conduite selon la méthode décrite dans l'exemple 2, mais en utilisant le N-oxyde de 10-N-deméthyl-10-N-(6'-phthalimidohexyl)-galanthamine à la place du N-oxyde de 10-N-deméthyl-10-N-(4-phthalimidobutyl)-galanthamine (rendement = 58 %).

RMN-¹H (300 MHz-EDCl₃): 8,95 (1H, s, H₉); 7,88-7,78 (4H, m, phthalimido), 7,69 (1H, d, H₈); 7,02 (1H, d, H₇); 6,15 (1H, dd, 2); 5,60 (1H, d, H₁); 4,78 (1H, large s, H₄ₐ); 4,25 (1H, large t, H₃); 4,19-4,02 (2H, m, H₁₁); 3,98 (3H, s, OCH₃); 3,66 (2H, t, 6'); 2,77 (1H, dm, H_{4.α}); 2,25 (2H, m, H_{1'}); 2,12 (1H, dm, H_{4.β}); 2,00-1,88 (2H, m, H_{5'}); 1,78-1,63 (3H, m, ,H_{12.α}, H_{2'}); 1,52-1,35 (5H, m, H_{3'}, H_{4'}, H_{12.β}).

### Exempte 4 : bromhydrate de 10-N-deméthyl-10-N-(8'-phthalimidooctyl)-galanthaminium

### Exemple 4a : synthèse indirecte

La réaction est conduite selon la méthode décrite dans l'exemple 2, mais en utilisant le N-oxyde de 10-N-deméthyl-10-N-(8'-phthalimidooctyl)galanthamine à la place du N-oxyde de 10-N-deméthyl-10-N-(4'-phthalimidobutyl)galanthamine.

### Exemple 4b : synthèse directe

A une solution de 72 mg (0,14 mmole) de 10-N-deméthyl-10-N-(8'-phthalimidooctyl)-galanthamine dans 2 ml de tétrachlorure de carbone, 32 mg (0,18 mmole, 1,3 éq) de N-bromosuccinimide et une quantité catalytique (5 %) d'azodiisobutyronitrile sont ajoutés. Le mélange est agité à température ambiante, sous argon et à l'abri de la lumière pendant 24 heures. 40 ml de dichlorométhane sont additionnés puis le mélange est lavé avec 20 ml d'une solution aqueuse saturée en chlorure de sodium. La phase organique est récupérée, séchée sur sulfate de sodium, filtrée et évaporée sous vide. Le résidu est purifié par chromatographie sur couche épaisse avec comme mélange éluant : dichlorométhane 90 / méthanol 9,9 / acide bromhydrique 0,5 % 0,1 (rendement = 52 %).

RMN-¹H (300 MHz-CDCl₃): 10,09 (1H, s, H₉); 8,03 ( 1 H, d, J=8.5 Hz, H₈); 7,83 (2H, m, H_{ar ortho}); 7,71 (2H, m, H_{ar meta}); 6,91 (1H, d, J=85 Hz, H₇); 6,19 (1H, dd, J₁ =10 Hz, J₂ =5 Hz, H₂); 5,81 (1H, d, J=10 Hz, H₁); 4,78 (1H, large s, H₄ₐ); 4,45-4,33 (3H, m, H_{1'}, H_{11.α}); 4,23 (1H, large s, H₃); 4,13 (1H, dm, J₁ =17 Hz, J₂ =4 Hz, H_{11.β}); 3,97 (3H, s, OCH₃); 3,66 (2H, t, J=7 Hz, H₁₀); 2,75 (1H, dm, J=16 Hz, H_{4.α}) 2,23 (2H, m, H₁₂); 2,09 (H, dm, J₁ =16 Hz, J₂ =5 Hz, H3 =2 Hz, H_{4.β}); 1,91 (2H, m, H_{2'}); 1,65 (2H, m, H_{9'}); 1,41(2H, m, H_{3'}); 1,30-1,26 (10H, m, H_{4'}, H_{5'}, H_{6'}, H_{7'}, H_{8'}).

### Exemple 5 : bromhydrate de 10-N-deméthyl-10-N-(10'-phthalimidodécyl)-galanthaminium

### Exemple 5a : synthèse indirecte

La réaction est conduite selon la méthode décrite dans l'exemple 2, mais en utilisant le N-oxyde de 10-N-deméthyl-10-N-(10'-phthalimidodécyl)galanthamine à la place du N-oxyde de 10-N-deméthyl-10-N-(4'-phthalimidobutyl)galanthamine.

### Exemple 5b : synthèse directe

En travaillant de la même manière que dans l'exemple 4b mais en utilisant la 10-N-deméthyl-10-N-(10'-phthalimidodécyl)-galanthamine à la place de la 10-N-deméthyl-10-N-(8'-phthalimidooctyl)-galanthamine, on obtient le composé recherché (rendement = 45 %).

RMN-¹H (300 MHz-CDCl₃): 10,17 (1H, s, H₉); 8,06 (1H, d, J=8.5 Hz, H₈); 7,83 (2H, m, H_{ar ortho}); 7,71 (2H, m, H_{ar meta}); 6,91 (1H, d, J=8.5 Hz, H₇); 6,19 (1H, dd, J₁ =10 Hz, J₂ =5 Hz, H₂); 5,81 (1H, d, J=10 Hz, H₁); 4,78 Hz(1H, large s, H₄ₐ); 4,45-4,33 (3H, m, H_{1'}, H_{11.α}); 4,23 (1H, large s, H₃); 4,13 (1H, dm, J₁ =17 Hz, J₂ =4 Hz, H_{11.β}); 3,97 (3H, s, OCH₃); 3,66 (2H, t, J=7 Hz, H_{10'}); 2,75 (1H, dm, J=16 Hz, H_{4.α}); 2,23 (2H, m, H₁₂); 2,09 (H, dm, J₁ =16 Hz, J₂ =5 Hz, J₃ =2 Hz, H_{4.β}); 1,91 (2H, m, H_{2'}); 1,65 (2H, m, H_{9'}); 1,41 (2H, m, H_{3'}); 1,30-1,26 (10H, m, H_{4'}, H_{5'}, H_{6'}, H_{7'}, H_{8'}).

### Exemple 6 : bromhydrate de 10-N-deméthyl-10-N-(12'-phthalimidododécyl)-galanthaminium

### Exemple 6a : synthèse indirecte

La réaction est conduite selon la méthode décrite dans l'exemple 2, mais en utilisant le N-oxyde de 10-N-deméthyl-10-N-(12'-phthalimidododécyl)galanthamine à la place du N-oxyde de 10-N-deméthyl-10-N-(4'-phthalimidobutyl)galanthamine.

### Exemple 6b : synthèse directe

A une solution de 73 mg (0,12 mmole) de 10-N-deméthyl-10-N-(12'-phthalimidododécyl)-galanthamine dans 5 ml d'éthanol absolu, 13 mg (0,16 mmole, 1,3 éq) d'acétate de sodium et 63 mg (0,25 mmole, 2 éq) d'iode sont additionnés. Le mélange est chauffé au reflux sous argon pendant 1 heure. 3 ml d'une solution de bisulfite de sodium à 10 % sont ajoutés goutte à goutte à température ambiante pour éliminer l'excès d'iode. Après évaporation de l'éthanol, le mélange est repris par 30 ml de dichlorométhane et lavé avec 50 ml d'eau. La phase organique est lavée successivement avec 50 ml d'une solution aqueuse saturée en carbonate de sodium et avec 50 ml d'une solution d'acide bromhydrique à 0,5 %. La phase organique est ensuite séchée sur sulfate de sodium, filtrée et évaporée sous vide. Le produit brut est chromatographié sur colonne de silice avec dichlorométhane 80 / éthanol 19 / acide bromhydrique 0,5 % l comme éluant (rendement = 28 %).

RMN-¹H (300 MHz-CDCl₃): 10,03 (1H, s, H₉); 8,03 (1H, d, J=8.5 Hz, H₈); 7,83 (2H, m, H_{ar ortho}); 7,71 (2H, m, H_{ar metha}); 6,90 (1H, d, J=8.5 Hz, H₇); 6,18 (1H, dd, J₁ =10 Hz, J₂ =5 Hz, H₂); 5,86 (1H, d, J=10 Hz, H₁); 4,78 (1H, large s, H₄ₐ); 4,47-4,34 (3H, m, H_{1'}, H_{11.α}); 4,22 (1H, large s, H₃); 4,18 (1H, dm, J₁ =17 Hz, J₂ =4 Hz, H_{11.β}); 3,96 (3H, s, OCH₃); 3,66 (2H, t, J=7 Hz, H_{12'}); 2,74 (1H, dm, J=16 Hz, H_{4.α}); 2,39 (1H, large s, OH); 2,23 (2H, m, H₁₂); 2,10 (H, dm, J₁ =16 Hz, J₂ =5 Hz, J₃ =2 Hz, H_{4.β}); 1,91 (2H, m, H_{2'}); 1,66 (2H, m, H_{11'}); 1,40-1,30 (4H, m, H_{3'}, H_{10'}); 1,23 (12H, large s, H_{4'}, H_{5'}, H_{6'}, H_{7'}, H_{8'}, H_{9'}).

### Exemple 7 : bromhydrate de 6-O-deméthyl-6-O-(8'phthalimidooctyl)-galanthaminium

A une solution de 64 mg (0,12 mmole) de 6-O-deméthyl-6-O-(8'-phthalimidooctyl)-galanthamine dans 4 ml d'éthanol absolu, 13 mg (0,16 mmole, 1,3 éq) d'acétate de sodium et 62 mg (0,24 mmole, 2 éq) d'iode sont additionnés. Le mélange est chauffé au reflux sous argon pendant 1 heure. 1 ml d'une solution de bisulfite de sodium à 10 % est ajoute goutte à goutte à température ambiante, pour éliminer l'excès d'iode. Après évaporation de l'éthanol, le mélange est repris par 60 ml de dichlorométhane et lavé avec 50 ml d'eau. La phase organique est lavée successivement avec 50 ml d'une solution d'acide bromhydrique à 0,5 %. La phase organique est ensuite séchée sur sulfate de sodium, filtrée et évaporée sous vide. Le produit brut est chromatographié sur colonne de silice avec dichlorométhane 90 / éthanol 9,9 / acide bromhydrique 0,5 % 0,1 comme éluant (rendement = 57 %).

RMN-¹H (300 MHz-CDCl₃ +2 gouttes CD₃ OD): 9,40 (1H, s, H₉); 7,84 (2H, m, Hₐᵣ ₒᵣₜₕₒ); 7,77 (1H, d, J=9 Hz, H₈); 7,73 (2H, m, H_{ar meta}); 6,96 (1 H, d, J=9 Hz, H₇); 6,16 (1H, dd, J₁ =10 Hz, J₂ =5 Hz, H₂); 5,79 (1H, d, J=10 Hz, H₁); 4,77 (1H, large s, H₄ₐ); 4,32 (1H, large t, J₁ =17 Hz, J₂ =13 Hz, H_{11.α}); 4,22 (1H, large t, J=5 Hz, H₃); 4,15-4,09 (3H, m, H_{1'}, H_{11.β}); 4,02 (3H, s, NCH₃); 3,68 (2H, t, J=7 Hz, H_{8'}); 2,76 (1H, dm, J=16 Hz, H_{4.α}); 2,30 (1H, tm, J₁ =15 Hz, J₂ =13 Hz, J₃ =3 Hz, H_{12.α}); 2,16 (1H, dm, J₁ =15 Hz, H_{12.β}); 2,10 (1H, dm, J₁ =16 Hz, J₂ =5.5 Hz, J₃ =2 Hz, H_{4.β}); 1,83 (2H, m, J=7 Hz, H_{2'}); 1,68 (2H, m, H_{7'}); 1,43 (6H, large s, H_{4'}, H_{5'}, H_{6'}).

### Exemple 8 : bromhydrate de 6-O-deméthyl-6-O-(4'-phthalimidobutyl)-galanthaminium

En travaillant de la même manière que dans l'exemple 7 mais en utilisant 6-O-deméthyl-6-O-(4'-phthalimidobutyl)-galanthamine à la place de la 6-O-deméthyl-6-O-(8'-phthalimidooctyl)-galanthamine, on obtient le produit recherché (rendement = 55 %).

RMN-¹H (300 MHz-CDCl₃ +2 gouttes CD₃ OD): 9,40 (1H, s, H₉); 7,84 (2H, m, Hₐᵣ ₒᵣₜₕₒ); 7,77 (1H, d, J=9 Hz, H₈); 7,73 (2H, m, H_{ar meta}); 6,96 (1 H, d, J=9 Hz, H₇); 6,16 (1H, dd, J₁ =10 Hz, J₂ =5 Hz, H₂); 5,79 (1H, d, J=10 Hz, H₁); 4,77 (1H, large s, H₄ₐ); 4,32 (1H, large t, J₁ =17 Hz, J₂ =13 Hz, H_{11.α}); 4,22 (1H, large t, J=5 Hz, H₃); 4,15-4,09 (3H, m, H_{1'}, H_{11.β}); 4,02 (3H, s, NCH₃); 3,68 (2H, t, J=7 Hz, H_{8'}); 2,76 (1H, dm, J=16 Hz, H_{4.α}); 2,30 (1H, tm, J₁ =15 Hz, J₂ =13 Hz, J₃ =3 Hz, H_{12.α}); 2,16 (1H, dm, J₁ =15 Hz, H_{12.β}); 2,10 (1H, dm, J₁ =16 Hz, J₂ =5.5 Hz, J₃ =2 Hz, H_{4.β}); 1,83 (2H, m, J=7 Hz, H_{2'}); 1,68 (2H, m, H_{7'}); 1,43 (6H, large s, H_{4'}, H_{5'}, H_{6'}).

### Exemple 9 : bromhydrate de 6-O-deméthyl-6-O-(10'-phthalimidodécyl)-galanthaminium

En travaillant de la même manière que dans l'exemple 7 mais en utilisant 6-O-deméthyl-6-O-(10'-phthalimidodécyl)-galanthamine à la place de la 6-O-deméthyl-6-O-(8'-phthalimidooctyl)-galanthamine, on obtient le produit recherché (rendement = 56%).

RMN-¹H (300 MHz-CDCl₃ +2 gouttes CD₃ OD): 9,44 (1H, s, H₉); 7,84 (2H, m, Hₐᵣ ₒᵣₜₕₒ); 7,78 (1H, d, J=8.5 Hz, H₈); 7,72 (2H, m, H_{ar meta}); 6,95 (1H, d, J=8.5 Hz, H₇); 6,17 (1H, dd, J₁ =10 Hz, J₂ =5 Hz, H₂); 5,80 (1H, d, J=10 Hz, H₁); 4,77 (1H, large s, H₄ₐ); 4,33 (1H, large t, J₁ =17 Hz, J₂ =13 Hz, H_{11.α}); 4,22 (1H, large t, J=5 Hz, H₃); 4,15-4,02 (3H, m, H_{1'}, H_{11.β}); 4,03 (3H, s, NCH₃); 3,67 (2H, t, J=7 Hz, H_{10'}); 2,74 (1H, dm, J=16 Hz, H_{4.α}); 2,30 (1H, tm, J₁ =15 Hz, J₂ =12 Hz, J₃ =3 Hz, H_{12.α}); 2,16 (1H, dm, J₁ =15 Hz, H_{12.β}); 2,10 (1H, dm, J₁ =16 Hz, J₂ =5 Hz, J₃ =2 Hz, H_{4.β}); 1,83 (2H, m, J=7 Hz, H_{2'}); 1,67 (2H, m, H_{9'}); 1,42 (2H, m, H_{3'}); 1,30 (10H, large s, H_{4'}, H_{5'}, H_{6'}, H_{7'}, H_{8'}).

### Exempte 10 : bromhydrate de 6-O-deméthyl-6-O-(12'-phthalimidododécyl)-galanthaminium

En travaillant de la même manière que dans l'exemple 7 mais en utilisant 6-O-deméthyl-6-O-(12'-phthalimidododécyl)-galanthamine à la place de la 6-O-deméthyl-6-O-(8'-phthalimidooctyl)-galanthamine, on obtient le produit recherché (rendement = 58 %).

RMN-¹H (300 MHz-CDCl₃ +2 gouttes CD₃ OD): 9,46 (1H, s, H₉); 7,85 (2H, m, Hₐᵣ ₒᵣₜₕₒ); 7,76 (1H, d, J=8.5 Hz, H₈); 7,73 (2H, m, H_{ar meta}); 6,95 (1H, d, J=8.5 Hz, H₇); 6,18 (1H, dd, J₁ =10 Hz, J₂ =4.5 Hz, H₂); 5,78 (1H, d, J=10 Hz, H₁); 4,75 (1H, large s, H₄ₐ); 4,31 (1H, large t, J₁ =17 Hz, J₂ =13 Hz, H_{11.α}); 4,22 (1H, large t, J=4.5 Hz, H₃); 4,18-4,07 (3H, m, H_{1'}, H_{11.β}); 4,03 (3H, s, NCH₃); 3,68 (2H, t, J=7 Hz, H_{12'}); 2,76 (1H, dm, J=16 Hz, H_{4.α}); 2,68 (1H, m, H_{12.α}); 2,28 (1H, dm, J=15 Hz, H_{12.β}); 2,10 (1H, dm, J=16 Hz, H_{4.β}); 1,85 (2H, m, J=7 Hz, H_{2'}); 1,68 (2H, m, H_{11'}); 1,31 (16H, large s, H_{3'}, H_{4'}, H_{5'}, H_{6'}, H_{7'}, H_{8'}, H_{9'}, H_{10'}).

### Exemple 11 : bromhydrate de 10-N-deméthyl-10-N-(6'-pyrrolohexyl)-galanthaminium

La réaction est conduite selon la méthode décrite dans l'exemple 1, mais en utilisant la 10-N-deméthyl-10-N-(6'-pyrrolohexyl)galanthamine à la place de la galanthamine (rendement = 58 %).

RMN-¹H (300 MHz-CDCl₃): 8,88 (1H, s, H₉); 8,86 (1H, large s, NH); 7,59 (1H, d, J=8.5 Hz, H₈); 7,23 (2H, t, J=2 Hz, H_{8'} et H_{11'}), 7,14 (2H, t, J=2 Hz, H_{9'}, H_{10'}); 7,00 (1H, d, J=8.5 Hz, H₇); 6,13 (1H, dd, J₁ =10 Hz, J₂ =5 Hz, H₂); 5,58 (1H, d, J=10 Hz, H₁); 4,80 (1H, large s, H₄ₐ); 4,32.gtoreq.4,23 (2H, m, H_{1'}); 4,15 (1H, large t, J=5 Hz, H₃); 4,12-4,00 (4H, m, H₁₁, H_{6'}); 3,99 (3H, s, OCH₃); 2,74 (1H, dm, J=16 Hz, H_{4.α}); 2,28-2,19 (2H, m, H₁₂); 2,11 (1H, ddd, J₁ =16 Hz, J₂ =5 Hz, J₃ =2 Hz, H_{4.β}); 2,00-1,88 (2H, m, H_{5'}); 1,82-1,68 (2H, m, H_{2'}); 1,49-1,36 (4H, m, H_{4'}, H_{3'}).

En utilisant les procédés indiqués ci-dessus, on peut également préparer les produits suivants, qui font également partie de l'invention et qui constituent des produits préférés :

| Composé | R-A- | R₆ | R₉ |
|---|---|---|---|
| B | méthyle | H | H |
| C | méthyle | méthyle | méthyle |
| D | pentyle | H | méthyle |
| E | octyle | H | H |
| F | décyle | éthyle | H |
| G | aminométhyle | H | H |
| H | aminobutyle | H | H |
| I | aminooctyle | propyle | éthyle |
| J | CH₃NH(CH₂)₄ | H | trifluorométhyle |
| K | C₂H₅NH(CH₂)₄ | propyle | hydroxybutyle |
| L | (CH₃)₃N⁺(CH₂)₄ | H | H |
| M | (CH₃)₃N⁺(CH₂)₆ | éthyle | H |
| N | phthalimidobutyle | H | H |
| O | phthalimidohexyle | H | H |
| P | phthalimidooctyle | H | formyle |
| Q | H | phthalimidobutyle | H |
| R | H | hexyle | acétyle |
| S | phthalimidodécyle | H | H |
| T | phthalimidododécyle | H | méthyle |
| U | méthyle | phthalimidobutyle | éthoxy |
| V | propyle | phthalimidohexyle | H |
| W | CH₃NH(CH₂)₄ | phthalimidooctyle | allyle |
| X | aminométhyle | phthalimidododécyle | méthoxycarbonyle |
| Y | cyanoaminométhyle | aminobutyle | H |
| Z | H | aminométhyle | cyclohexyléthyle |
| AA | H | aminooctyle | hydroxyméthyle |
| AB | méthyle | aminododécyle | H |
| AC | aminodécyle | éthyle | cyanométhyle |
| AD | (1-pyrrolyle)octyle | méthyle | H |
| AE | (1-pyrrolyle)butyle | H | aminoéthyle |
| AF | benzylaminoéthyle | méthyle | H |
| AG | (2-MeO.C₆H₄)C₂H₄ | méthyle | H |
| AH | CH₃C(O)C₂H₄ | H | H |
| AI | CH₃C(O)C₃H₆ | méthyle | vinyle |
| AJ | méthyle | méthyle | allyle |
| AK | méthyle | méthyle | vinyle |
| AL | méthyle | méthyle | diméthylaminométhyle |
| AM | (CH₃)₃N⁺(CH₂)₄ | méthyle | H |
| AN | (CH₃)₃N⁺(CH₂)₆ | méthyle | H |
| AO | thioéthyle | méthyle | H |
| AP | aminoéthyle | méthyle | H |
| AQ | hydroxyéthyle | méthyle | H |
| AR | thiopropyle | méthyle | H |
| AS | thiobutyle | méthyle | H |
| AT | aminopropyle | méthyle | H |
| AU | aminobutyle | méthyle | H |
| AV | hydroxypropyle | méthyle | H |
| AW | hydroxybutyle | méthyle | H |
| AX | méthyle | méthyle | diméthylaminoéthyle |
| AY | méthyle | méthyle | diméthylaminopropyle |
| AZ | méthyle | méthyle | diméthylaminobutyle |
| BA | méthyle | méthyle | morpholinoéthyle |
| BB | méthyle | méthyle | trifluoroacétyle |
| BC | phthalimidooctyle | méthyle | vinyle |
| BD | phthalimidooctyle | méthyle | allyle |
| BE | phthalimidooctyle | H | vinyle |
| BF | phthalimidooctyle | H | allyle |
| BG | phthalimido-oct-4-ényle | méthyle | H |
| BH | phthalimido-oct-4-ynyle | méthyle | H |

### Etude pharmacologique des produits de l'invention

Afin de mesurer l'activité inhibitrice de cholinestérase des composés de l'invention, on évalue l'activité enzymatique de l'acétylcholinestérase par la méthode d'Ellman (Biochemical Pharmacology, 1961, pp 88-95, Ellman et coll.).

Le principe de la méthode est de mesurer le taux de thiocholine lors de l'hydrolyse de l'acétylcholine par l'enzyme. L'hydrolyse s'accompagne d'une réaction continue entre la thiocholine et l'acide 5,5'-dithio-2-nitrobenzoïque (DTNB) qui conduit à la formation de l'anion 5-thio-2-nitrobenzoate de couleur jaune. Le taux de formation de l'anion est mesuré par absorbance à 412 nm.

Le matériel utilisé comprend un tampon phosphate : Na₂HPO₄/NaH₂PO₄ 0,1 M (pH 8), une enzyme : l'acétylcholinestérase de l'organe électrique de torpille Electrophorus *electricus* (SIGMA C 2888) purifiée sur résine de Sephadex, un substrat : l'iodure d'acétylthiocholine 7,5 mM (21,67 mg / 10 ml de tampon phosphate) et un réactif : l'acide 5,5'-dithio-2-nitrobenzoïque (DTNB) 10 mM (39,6 mg / 10 ml de tampon phosphate).

Ainsi 3 ml de tampon phosphate, 100 µl de DTNB, 2 µl d'enzyme, 3,3 µl de composé selon l'invention, et 200 µl de substrat sont mélangés à 25°C puis le changement d'absorbance à 412 nm est immédiatement enregistré. Les résultats obtenus, exprimés en concentration d'inhibiteur, sont résumés dans le tableau ci-dessous (IC₅₀ représente la concentration inhibant à 50 % l'activité de l'enzyme).

| Exemple | IC₅₀ ± SD (10⁻⁷M) |
|---|---|
| 1 | 0,8 ± 0,02 |
| 2 | 4,7 ± 0,4 |
| 3 | 0,4 ± 0,01 |
| 4 | 0,1 ± 0,02 |
| 5 | 0,2 ± 0,01 |
| 6 | 1,3 ± 0,09 |
| 7 | 0,7 ± 0,1 |
| 9 | 0,5 ± 0,09 |
| 10 | 3,2 ± 0,9 |
| 11 | 1,7 ± 0,1 |

## Revendications

1. Les composés de formule générale la 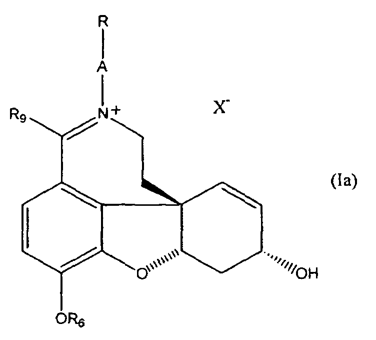 et Ib 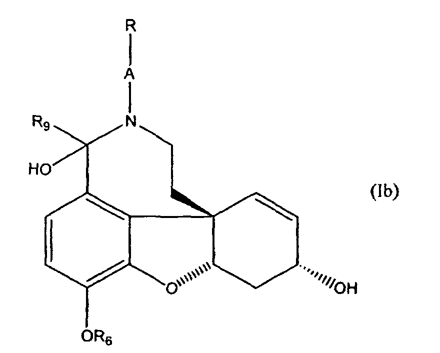 dans laquelle:
A représente un group alkylène linéaire ou ramifié, saturé ou insaturé, contenant de 1 à 12 atomes de carbone ;
R représente un atome d'hydrogène ou un groupe de formule -NR'R" ou -N⁺R'R''R''' dans laquelle
R' et R" représentent, indépendamment un atome d'hydrogène ; un radical cyano; un radical alkyle comprenant de 1 à 12 atomes de carbone ; un radical aryle lié à un groupe alkyle comprenant de 1 à 12 atomes de carbone, le radical aryle étant choisi parmi les radicaux phényle, naphtyle, thiényle, furyle, pyrrolyle, imidazolyle, pyrazolyle, isothiazole, thiazole, isoxazolyle, oxazole, pyridyle, pyrazyle, pyrimidyle, benzothiényle, benzofuryle et indolyle; ou R' et R" sont liés entre eux et forment avec l'atome d'azote auquel ils sont rattachés un hétérocycle choisi parmi les radicaux pyrrole, imidazole, isothiazole, thiazole, isoxazole, oxazole, pyridine, pyrazine, pyrimidine, pyridazine, indole, isoindole, indazole, quinoline, isoquinoline, phthalazine, quinazoline, pyrrolidine, imidazolidine, pyrrazolidine, piperidine, piperazine, morpholine, thiazolidine et phthalimide, R''' représente, un radical cyano, un radical alkyle comprenant de 1 à 12 atomes de carbone,
R₆ représente un atome d'hydrogène ou un radical de formule -A-R dans laquelle A et R ont la signification indiquée ci-dessus ;
R₉ représente un atome d'hydrogène ou un radical de formule R'₉ dans laquelle R'₉ représente un radical alkyle linéaire ou ramifié, contenant de 1 à 12 atomes de carbone ou un radical alcènyle linéaire ou ramifié, contenant de 1 à 12 atomes de carbone;
X représente un anion pharmaceutiquement acceptable.

2. Les composés de formule générale telle que définie dans la revendication 1 et répondant aux formules suivantes :
- le méthanesulfonate de galanthaminium ;
- le trifluoroacétate de10-N-déméthyl-10-N-(4'-phthalimidobutyl)-galanthaminium ;
- le trifluoroacétate de 10-N-déméthyl-10-N-(6'-phthalimidohexyl)-galanthaminium ;
- le trifluoroacétate de 10-N-déméthyl-10-N-(8'-phthalimidooctyl)-galanthaminium ;
- le bromohydrate de 10-N-déméthyl-10-N-(10'-phthalimidodécyl)-galanthaminium ;
- le bromohydrate de 10-N-déméthyl-10-N-(12'-phthalimidododécyl)-galanthaminium ;
- le bromohydrate de 6-O-déméthyl-6-O-(8'-phthalimidooctyl)-galanthaminium ;
- le bromohydrate de 6-O-déméthyl-6-O-(4'-phthalimidobutyl)-galanthaminium ;
- le bromohydrate de 6-O-déméthyl-6-O-(10'-phthalimidodécyl)-galanthaminium ;
- le bromohydrate de 6-O-déméthyl-6-O-(12'-phthalimidododécyl)-galanthaminium ;
- le bromohydrate de 10-N-déméthyl-10-N-(6'-pyrrolohexyl)-galanthaminium.

3. Procédé de préparation des produits de formules générales Ia et Ib telles que définies à la revendication 1, **caractérisé en ce que** :
a) soit l'on oxyde directement un composé de formule générale (1a) 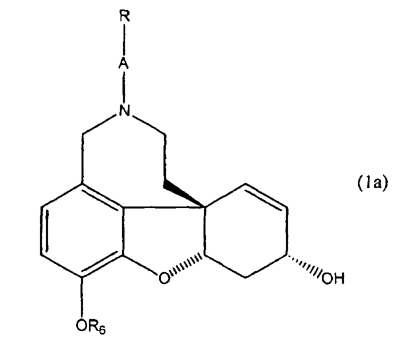 dans laquelle R, A et R₆ ont la signification indiquée à la revendication 1 ;
b) soit l'on transforme le composé de formule (1b) telle que définie ci-dessus, en son N-oxyde de formule (2a) 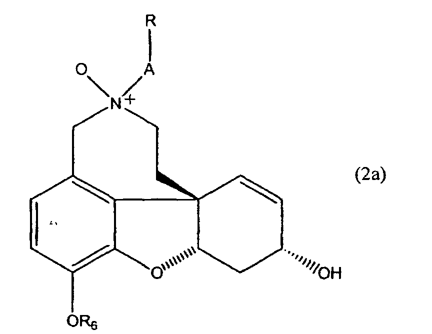 dans laquelle R, A et R₆ ont la signification indiquée à la revendication 1, produit de formule (2a) que l'on fait réagir avec un anhydride d'acide, sous atmosphère inerte, dans un solvant inerte, à une température comprise entre 0°C et la température ambiante,
pour obtenir un produit de formule Ia ou Ib dans laquelle R₉ représente un atome d'hydrogène, et
si le produit de formule Ia ou Ib dans laquelle R₉ représente R'₉ est recherché,
l'on traite le produit correspondant ainsi obtenu de formule Ia dans laquelle R₉ représente un atome d'hydrogène, avec un produit représentant une fonction nucléophile de formule R'₉Y dans laquelle R'₉ a la signification indiquée à la revendication 1 et Y représente un radical approprié, pour obtenir le composé de formule (1b) 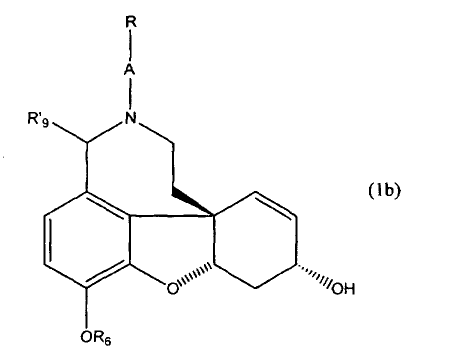 dans laquelle R, A, R₆ et R'₉ ont la signification indiquée à la revendication 1, puis
c) soit l'on oxyde directement le composé de formule générale (1b),
d) soit l'on transforme le composé de formule générale (1b) en son N-oxyde de formule (2b) 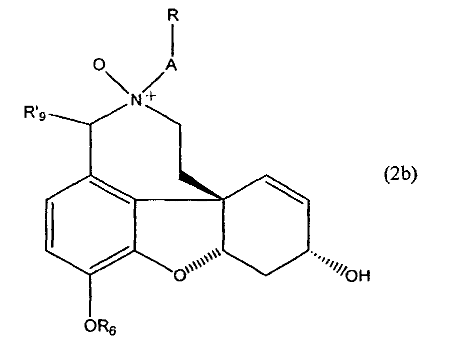
dans laquelle R, A, R₆ et R'₉ ont la signification indiquée à la revendication 1, produit de formule (2b) que l'on fait réagir avec un anhydride d'acide, sous atmosphère inerte, dans un solvant inerte, à une température comprise entre 0°C et la température ambiante,
pour obtenir un produit de formule Ia ou Ib dans laquelle R₉ représente R'₉.

4. A titre de médicaments, les produits de formule Ia et Ib telles que définies à la revendication 1, ainsi que les sels d'addition avec les acides minéraux ou organiques pharmaceutiquement acceptables desdits produits de formule Ib.

5. A titre de médicaments, les produits de formule Ia et Ib telles que définies dans la revendication 2, ainsi que les sels d'addition avec les acides minéraux ou organiques pharmaceutiquement acceptables desdits produits de formule Ib.

6. Compositions pharmaceutiques contenant, à titre de principe actif, l'un au moins des médicaments tels que définis à l'une des revendications 4 ou 5.

7. Utilisation des produits de formule Ia et Ib telles que définies à l'une quelconque des revendications 1 et 2 pour la préparation de médicaments destinés à traiter les maladies neurodégénératives.

8. Utilisation des produits de formule Ia et Ib telles que définies à l'une quelconque des revendications 1 et 2 pour la préparation de médicaments destinés à traiter les démences séniles.

9. A titre de produits industriels nouveaux, les composés de formule (1a), (1b), (2a) et (2b) 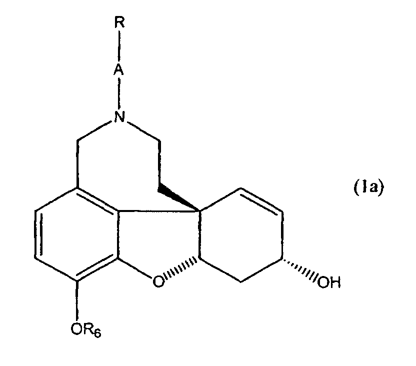 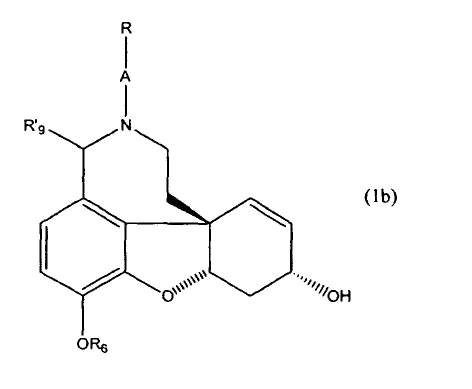 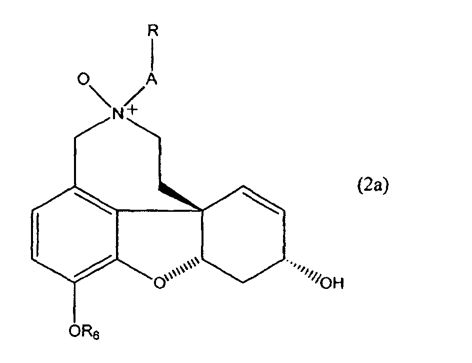 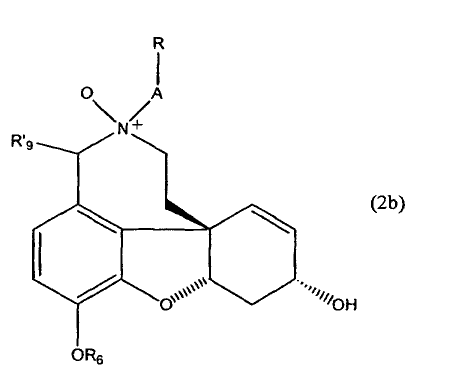 dans lesquels
A représente un group alkylène linéaire ou ramifié, saturé ou insaturé, contenant de 1 à 12 atomes de carbone ;
R représente un groupe de formule -NR'R" ou -N⁺R'R''R''' dans laquelle
R' et R" représentent, indépendamment un atome d'hydrogène ; un radical cyano; un radical alkyle comprenant de 1 à 12 atomes de carbone ; un radical aryle lié à un groupe alkyle comprenant de 1 à 12 atomes de carbone, le radical aryle étant choisi parmi les radicaux phényle, naphtyle, thiényle, furyle, pyrrolyle, imidazolyle, pyrazolyle, isothiazole, thiazole, isoxazolyle, oxazole, pyridyle, pyrazyle, pyrimidyle, benzothiényle, benzofuryle et indolyle; ou R' et R" sont liés entre eux et forment avec l'atome d'azote auquel ils sont rattachés un hétérocycle choisi parmi les radicaux pyrrole, imidazole, isothiazole, thiazole, isoxazole, oxazole, pyridine, pyrazine, pyrimidine, pyridazine, indole, isoindole, indazole, quinoline, isoquinoline, phthalazine, quinazoline, pyrrolidine, imidazolidine, pyrrazolidine, piperidine, piperazine, morpholine, thiazolidine et phthalimide, R''' représente, un radical cyano, un radical alkyle comprenant de 1 à 12 atomes de carbone,
R₆ représente un atome d'hydrogène ou un radical de formule -A-R dans laquelle A et R ont la signification indiquée ci-dessus et
R'₉ représente un radical alkyle linéaire ou ramifié, contenant de 1 à 12 atomes de carbone ou un radical alcènyle linéaire ou ramifié, contenant de 1 à 12 atomes de carbone.

## Patentansprüche

1. Verbindungen der allgemeinen Formel la 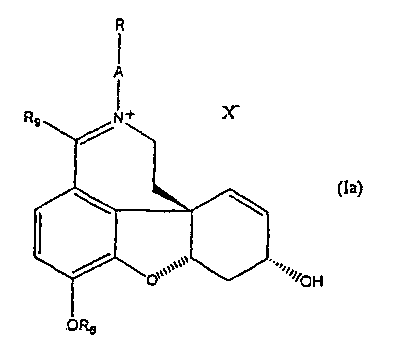 und Ib 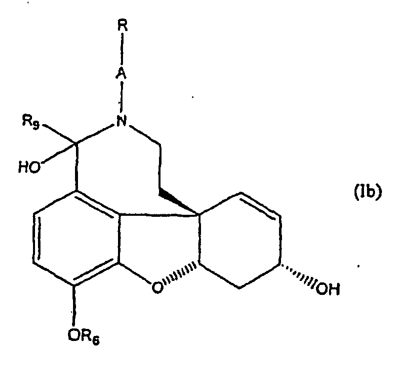 in denen:
A eine lineare oder verzweigte, gesättigte oder ungesättigte Alkylengruppe darstellt, die 1 bis 12 Kohlenstoffatome enthält;
R ein Wasserstoffatom oder eine Gruppe der Formel -NR'R" oder -N⁺R'R''R''' darstellt, in der
R' und R" unabhängig ein Wasserstoffatom; einen Cyanorest; einen Alkylrest, der 1 bis 12 Kohlenstoffatome umfasst; einen Arylrest, der an eine Alkylgruppe gebunden ist, die 1 bis 12 Kohlenstoffatome umfasst, darstellen, wobei der Arylrest aus Phenyl-, Naphthyl-, Thienyl-, Furyl-, Pyrrolyl-, Imidazolyl-, Pyrazolyl-, Isothiazol-, Thiazol-, Isoxazolyl-, Oxazol-, Pyridyl-, Pyrazyl-, Pyrimidyl-, Benzothienyl-, Benzofuryl- und Indolylresten ausgewählt ist; oder R' und R" miteinander verbunden sind und mit dem Stickstoffatom, an das sie geknüpft sind, einen Heterocyclus bilden, der aus Pyrrol-, Imidazol-, Isothiazol-, Thiazol-, Isoxazol-, Oxazol-, Pyridin-, Pyrazin-, Pyrimidin-, Pyridazin-, Indol-, Isoindol-, Indazol-, Chinolin-, Isochinolin-, Phthalazin-, Chinazolin-, Pyrrolidin-, Imidazolidin-, Pyrazolidin-, Piperidin-, Piperazin-, Morpholin-, Thiazolidin- und Phthalimidresten ausgewählt ist; R''' einen Cyanorest, einen Alkylrest darstellt, der 1 bis 12 Kohlenstoffatome umfasst;
R₆ ein Wasserstoffatom oder einen Rest der Formel -A-R darstellt, in der A und R die oben angegebene Bedeutung aufweisen;
R₉ ein Wasserstoffatom oder einen Rest der Formel R'₉ darstellt, in der R'₉ einen linearen oder verzweigten Alkylrest, der 1 bis 12 Kohlenstoffatome enthält, oder einen linearen oder verzweigten Alkenylrest darstellt, der 1 bis 12 Kohlenstoffatome enthält;
X ein pharmazeutisch annehmbares Anion darstellt.

2. Verbindungen der allgemeinen Formel wie in Anspruch 1 definiert und den folgenden Formeln entsprechend:
- Galanthaminiummethansulfonat;
- 10-N-Desmethyl-10-N-(4'-phthalimidobutyl)galanthaminiumtrifluoracetat;
- 10-N-Desmethyl-10-N-(6'-phthalimidohexyl)galanthaminiumtrifluoracetat;
- 10-N-Desmethyl-10-N-(8'-phthalimidooctyl)galanthaminiumtrifluoracetat;
- 10-N-Desmethyl-10-N-(10'-phthalimidodecyl)galanthaminiumhydrobromid;
- 10-N-Desmethyl-10-N-(12'-phthalimidododecyl)galanthaminiumhydrobromid;
- 6-O-Desmethyl-6-O-(8'-phthalimidooctyl)galanthaminiumhydrobromid;
- 6-O-Desmethyl-6-O-(4'-phthalimidobutyl)galanthaminiumhydrobromid;
- 6-O-Desmethyl-6-O-(10'-phthalimidodecyl)galanthaminiumhydrobromid;
- 6-O-Desmethyl-6-O-(12'-phthalimidododecyl)galanthaminiumhydrobromid;
- 10-N-Desmethyl-10-N-(6'-pyrrolohexyl)galanthaminiumhydrobromid.

3. Verfahren zur Herstellung von Produkten der allgemeinen Formeln la und Ib, wie in Anspruch 1 definiert, **dadurch gekennzeichnet, dass**
a) man entweder direkt eine Verbindung der allgemeinen Formel (1a) 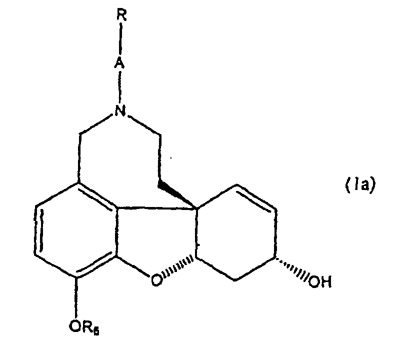 in der R, A und R₆ die in Anspruch 1 angegebene Bedeutung aufweisen, oxidiert;
b) oder die Verbindung der Formel (Ib), wie vorstehend definiert, in ihr N-Oxid der Formel (2a) 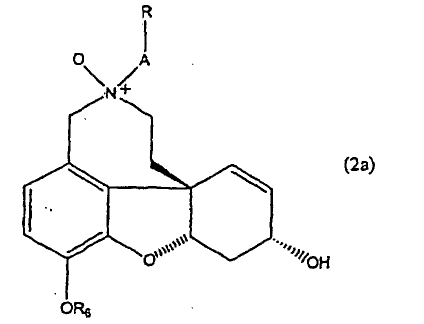 in der R, A und R₆ die in Anspruch 1 angegebene Bedeutung aufweisen, überführt, das Produkt der Formel (2a) mit einem Säureanhydrid unter Inertatmosphäre in einem inerten Lösungsmittel bei einer Temperatur zwischen 0°C und Umgebungstemperatur reagieren lässt,
um ein Produkt der Formel la oder Ib zu erhalten, in der R₉ ein Wasserstoffatom darstellt, und
wenn das Produkt der Formel la oder Ib gewünscht wird, in der R₉ für R'₉ steht, man das entsprechende so erhaltene Produkt der Formel la, in der R₉ ein Wasserstoffatom darstellt, mit einem Produkt behandelt, das eine nukleophile Funktion der Formel R'₉Y aufweist, in der R'₉ die in Anspruch 1 angegebenene Bedeutung aufweist und Y einen geeigneten Rest darstellt, um die Verbindung der Formel (1b) 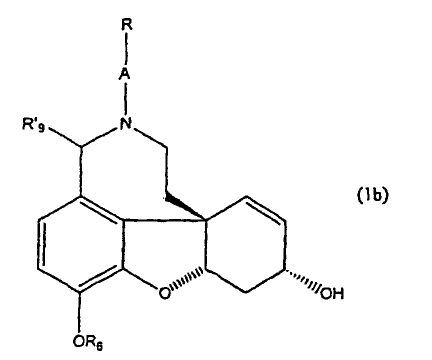 zu erhalten, in der R, A, R₆ und R'₉ die in Anspruch 1 angegebene Bedeutung aufweisen, dann
c) entweder direkt die Verbindung der allgemeinen Formel (1b) oxidiert,
d) oder die Verbindung der allgemeinen Formel (1b) in ihr N-Oxid der Formel (2b) 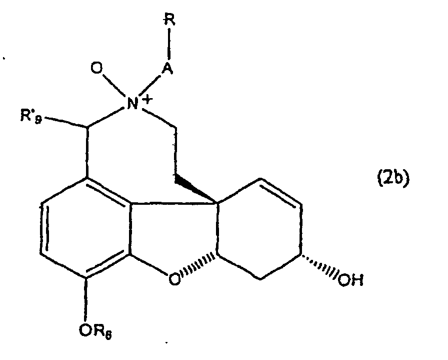 in der R, A, R₆ und R'₉ die in Anspruch 1 angegebene Bedeutung aufweisen, überführt, das Produkt der Formel (2b) mit einem Säureanhydrid unter Inertatmosphäre in einem inerten Lösungsmittel bei einer Temperatur zwischen 0°C und Umgebungstemperatur reagieren läasst, um ein Produkt der Formel la oder Ib zu erhalten, in der R₉ für R'₉ steht.

4. Produkte der Formel la und Ib, wie in Anspruch 1 definiert, sowie die Additionssalze der Produkte der Formel Ib mit pharmazeutisch annehmbaren Mineral- oder organischen Säuren als Medikamente.

5. Produkte der Formel la und lb, wie in Anspruch 2 definiert, sowie die Additionssalze der Produkte der Formel Ib mit pharmazeutisch annehmbaren Mineral- oder organischen Säuren als Medikamente.

6. Pharmazeutische Zusammensetzungen, die als Wirkstoff mindestens eines der Medikamente enthalten, wie in einem der Ansprüche 4 oder 5 definiert.

7. Verwendung der Produkte der Formel la und lb, wie in irgendeinem der Ansprüche 1 und 2 definiert, für die Herstellung von Medikamenten, die zur Behandlung von neurodegenerativen Krankheiten bestimmt sind.

8. Verwendung der Produkte der Formel la und Ib, wie in irgendeinem der Ansprüche 1 und 2 definiert, für die Herstellung von Medikamenten, die zur Behandlung von senilen Demenzen bestimmt sind.

9. Verbindungen der Formel (1a), (1b), (2a) und (2b) 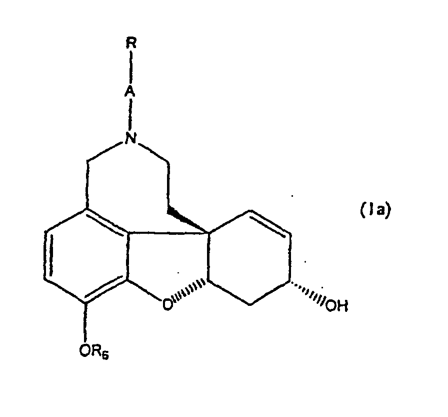 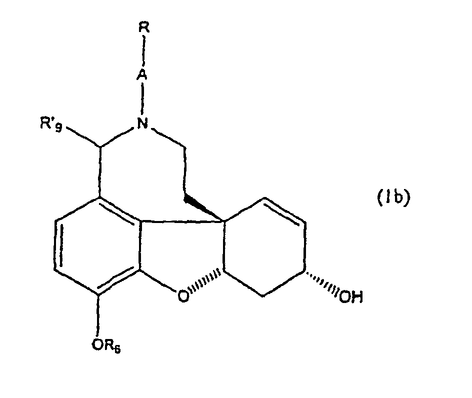 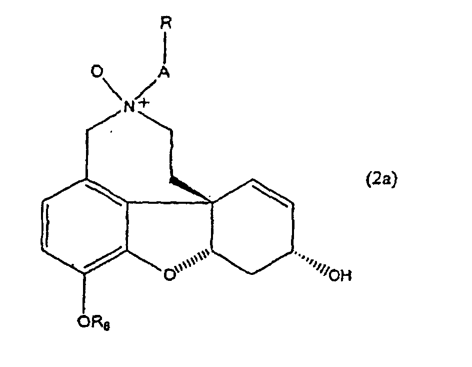 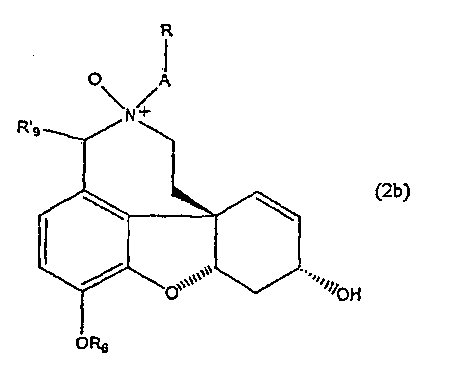 in denen
A eine lineare oder verzweigte, gesättigte oder ungesättigte Alkylengruppe darstellt, die 1 bis 12 Kohlenstoffatome enthält;
R eine Gruppe der Formel -NR'R" oder -N⁺R'R''R''' darstellt, in der
R' und R" unabhängig ein Wasserstoffatom; einen Cyanorest; einen Alkylrest, der 1 bis 12 Kohlenstoffatome umfasst; einen Arylrest, der an eine Alkylgruppe gebunden ist, die 1 bis 12 Kohlenstoffatome umfasst, darstellen, wobei der Arylrest aus Phenyl-, Naphthyl-, Thienyl-, Furyl-, Pyrrolyl-, Imidazolyl-, Pyrazolyl-, Isothiazol-, Thiazol-, Isoxazolyl-, Oxazol-, Pyridyl-, Pyrazyl-, Pyrimidyl-, Benzothienyl-, Benzofuryl- und Indolylresten ausgewählt ist; oder R' und R" miteinander verbunden sind und mit dem Stickstoffatom, an das sie geknüpft sind, einen Heterocyclus bilden, der aus Pyrrol-, Imidazol-, Isothiazol-, Thiazol-, Isoxazol-, Oxazol-, Pyridin-, Pyrazin-, Pyrimidin-, Pyridazin-, Indol-, Isoindol-, lndazol-, Chinolin-, Isochinolin-, Phthalazin-, Chinazolin-, Pyrrolidin-, Imidazolidin-, Pyrazolidin-, Piperidin-, Piperazin-, Morpholin-, Thiazolidin- und Phthalimidresten ausgewählt ist; R''' einen Cyanorest, einen Alkylrest darstellt, der 1 bis 12 Kohlenstoffatome umfasst:
R₆ ein Wasserstoffatom oder einen Rest der Formel -A-R darstellt, in der A und R die oben angegebene Bedeutung aufweisen; und
R'₉ einen linearen oder verzweigten Alkylrest, der 1 bis 12 Kohlenstoffatome enthält, oder einen linearen oder verzweigten Alkenylrest, der 1 bis 12 Kohlenstoffatome enthält, darstellt,
als neue industrielle Produkte.

## Claims

1. Compounds of general formula Ia 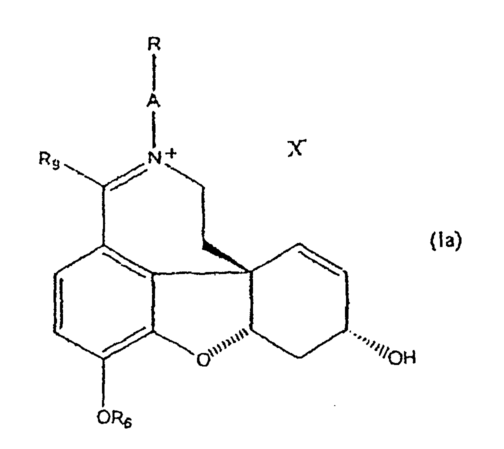 and Ib 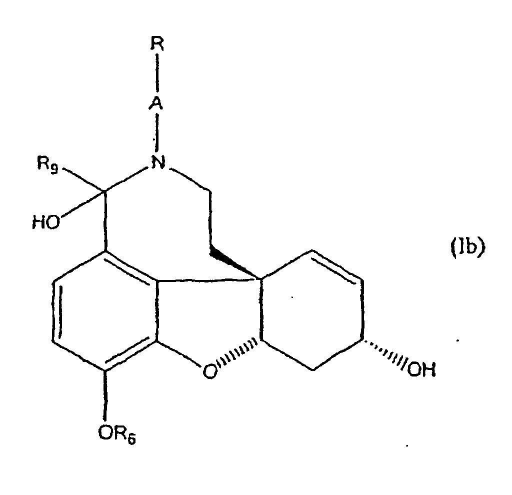 in which:
A represents a linear or branched, saturated or unsaturated alkylene group containing from 1 to 12 carbon atoms;
R represents a hydrogen atom or a group of formula -NR'R" or -N⁺R'R"R"' in which
R' and R" independently represent a hydrogen atom; a cyano radical; an alkyl radical containing from 1 to 12 carbon atoms; an aryl radical linked to an alkyl group containing from 1 to 12 carbon atoms, the aryl radical being chosen from phenyl, naphthyl, thienyl, furyl, pyrrolyl, imidazolyl, pyrazolyl, isothiazolyl, thiazolyl, isoxazolyl, oxazolyl, pyridyl, pyrazyl, pyrimidyl, benzothienyl, benzofuryl and indolyl radicals; or R' and R" are linked together and form, with the nitrogen atom to which they are attached, a heterocycle chosen from pyrrole, imidazole, isothiazole, thiazole, isoxazole, oxazole, pyridine, pyrazine, pyrimidine, pyridazine, indole, isoindole, indazole, quinoline, isoquinoline, phthalazine, quinazoline, pyrrolidine, imidazolidine, pyrazolidine, piperidine, piperazine, morpholine, thiazolidine and phthalimide radicals, and R"' represents a cyano radical or an alkyl radical containing from 1 to 12 carbon atoms,
R₆ represents a hydrogen atom or a radical of formula -A-R in which A and R have the meaning given above;
R₉ represents a hydrogen atom or a radical of formula R'₉ in which R'₉ represents a linear or branched alkyl radical containing from 1 to 12 carbon atoms or a linear or branched alkenyl radical containing from 1 to 12 carbon atoms;
X represents a pharmaceutically acceptable anion.

2. Compounds having the general formula as defined in Claim 1 and corresponding to the following formulae:
- galanthaminium methanesulphonate;
- 10-N-demethyl-10-N-(4'-phthalimidobutyl)galanthaminium trifluoroacetate;
- 10-N-demethyl-10-N-(6'-phthalimidohexyl)galanthaminium trifluoroacetate;
- 10-N-demethyl-10-N-(8'-phthalimidooctyl)galanthaminium trifluoroacetate;
- 10-N-demethyl-20-N-(10'-phthalimidodecyl)galanthaminium hydrobromide;
- 10-N-demethyl-10-N-(12'-phthalimidododecyl)galanthaminium hydrobromide;
- 6-O-demethyl-6-O-(8'-phthalimidooctyl)galanthaminium hydrobromide;
- 6-O-demethyl-6-O-(4'-phthalimidobutyl)galanthaminium hydrobromide;
- 6-O-demethyl-6-O-(10'-phthalimidodecyl)galanthaminium hydrobromide;
- 6-O-demethyl-6-O-(12'-phthalimidododecyl)galanthaminium hydrobromide;
- 10-N-demethyl-10-N-(6'-pyrrolohexyl)galanthaminium hydrobromide.

3. Process for preparing the products of general formulae Ia and Ib as defined in Claim 1, **characterized in that**:
a) either a compound of general formula (1a) 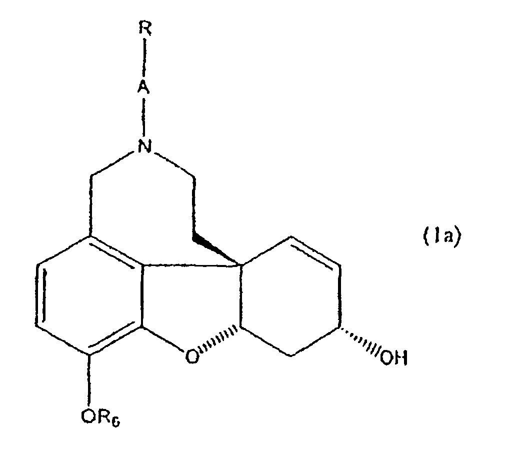 in which R, A and R₆ have the meaning given in Claim 1, is oxidized directly;
b) or the compound of formula (1b) as defined above is converted into its N-oxide of formula (2a) 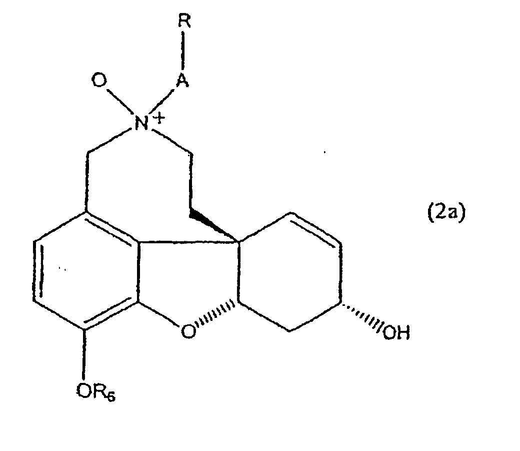 in which R, A and R₆ have the meaning given in Claim 1, and this product of formula (2a) is reacted with an acid anhydride, under an inert atmosphere, in an inert solvent, at a temperature of between 0°C and room temperature,
to give a product of formula Ia or Ib in which R₉ represents a hydrogen atom, and
if the product of formula Ia or Ib in which R₉ represents R'₉ is desired,
the corresponding product thus obtained of formula Ia, in which R₉ represents a hydrogen atom, is treated with a product representing a nucleophilic function of formula R'₉Y in which R'₉ has the meaning given in Claim 1 and Y represents a suitable radical, to obtain the compound of formula (1b) 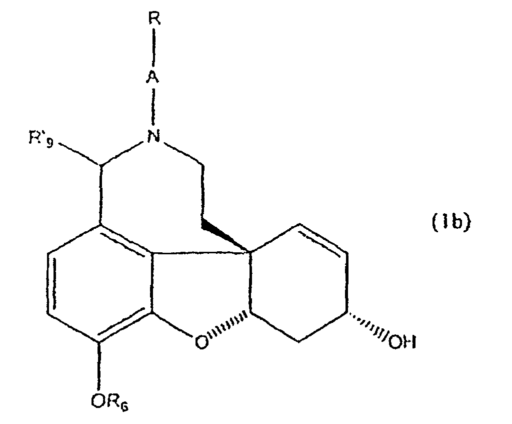 in which R, A, R₆ and R'₉ have the meaning given in Claim 1, and then
c) either the compound of general formula (1b) is oxidized directly,
d) or the compound of general formula (1b) is converted into its N-oxide of formula (2b) 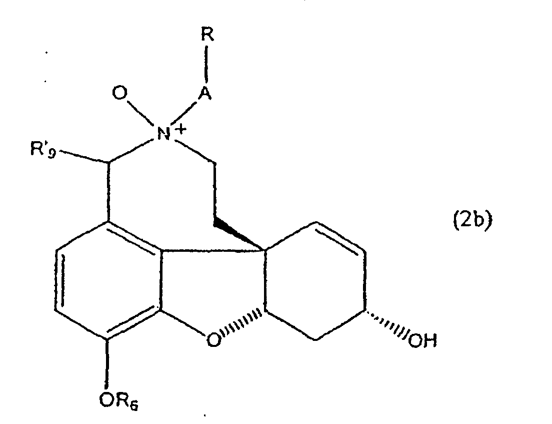
in which R, A, R₆ and R'₉ have the meaning given in Claim 1, and this product of formula (2b) is reacted with an acid anhydride, under an inert atmosphere, in an inert solvent, at a temperature of between 0°C and room temperature,
to give a product of formula Ia or Ib in which R₉ represents R'₉.

4. As medicinal products, the products of formulae Ia and Ib as defined in Claim 1, and also the addition salts with pharmaceutically acceptable mineral or organic acids of the said products of formula Ib.

5. As medicinal products, the products of formulae Ia and Ib as defined in Claim 2, and also the addition salts with pharmaceutically acceptable mineral or organic acids of the said products of formula Ib.

6. Pharmaceutical compositions containing, as active principle, at least one of the medicinal products as defined in either of Claims 4 and 5.

7. Use of the products of formulae Ia and Ib as defined in either of Claims 1 and 2, for the preparation of medicinal products for treating neurodegenerative diseases.

8. Use of the products of formulae Ia and Ib as defined in either of Claims 1 and 2, for the preparation of medicinal products for treating senile dementia.

9. As novel industrial products, the compounds of formulae (1a), (1b), (2a) and (2b) 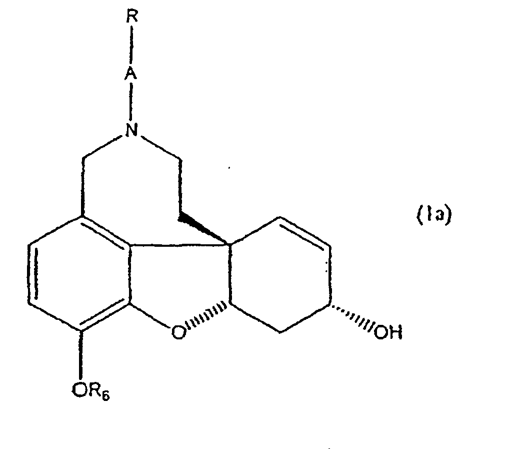 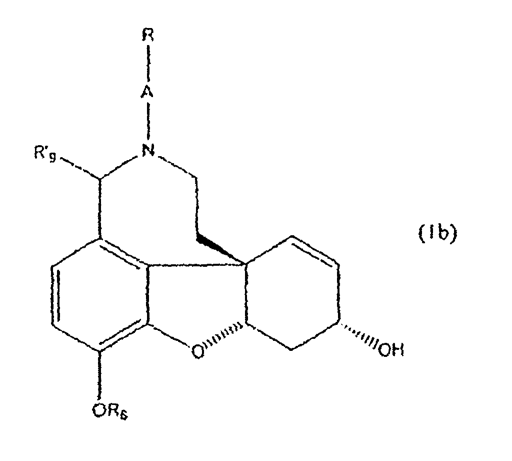 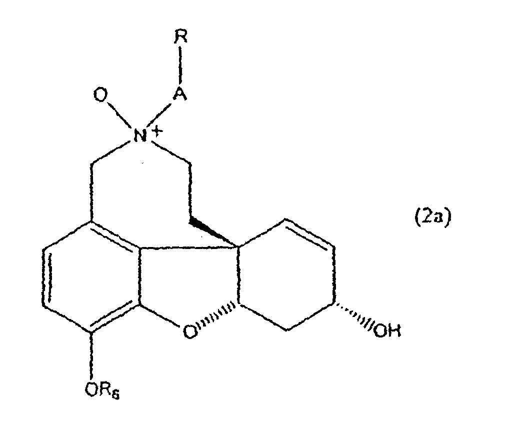 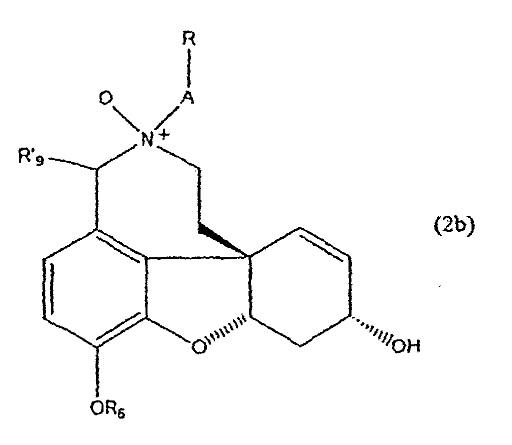 in which
A represents a linear or branched, saturated or unsaturated alkylene group containing from 1 to 12 carbon atoms;
R represents a group of formula -NR'R" or -N⁺R'R"R"' in which
R' and R" independently represent a hydrogen atom; a cyano radical; an alkyl radical containing from 1 to 12 carbon atoms; an aryl radical linked to an alkyl group containing from 1 to 12 carbon atoms, the aryl radical being chosen from phenyl, naphthyl, thienyl, furyl, pyrrolyl, imidazolyl, pyrazolyl, isothiazolyl, thiazolyl, isoxazolyl, oxazolyl, pyridyl, pyrazyl, pyrimidyl, benzothienyl, benzofuryl and indolyl radicals; or R' and R" are linked together and form, with the nitrogen atom to which they are attached, a heterocycle chosen from pyrrole, imidazole, isothiazole, thiazole, isoxazole, oxazole, pyridine, pyrazine, pyrimidine, pyridazine, indole, isoindole, indazole, quinoline, isoquinoline, phthalazine, quinazoline, pyrrolidine, imidazolidine, pyrazolidine, piperidine, piperazine, morpholine, thiazolidine and phthalimide radicals, and R"' represents a cyano radical or an alkyl radical containing from 1 to 12 carbon atoms,
R₆ represents a hydrogen atom or a radical of formula -A-R in which A and R have the meaning given above and
R₉ represents a hydrogen atom or a radical of formula R'₉ represents a linear or branched alkyl radical containing from 1 to 12 carbon atoms or a linear or branched alkenyl radical containing from 1 to 12 carbon atoms.
